# EUROPÄISCHE PATENTANMELDUNG

(11) **EP 1 902 727 A1**
(43) Veröffentlichungstag der Anmeldung: **26.03.2008**
(21) Anmeldenummer: 06019910.6
(22) Anmeldetag: 22.09.2006
(51) Int. Cl.: A61K 39/39, A61K 39/29

(54) **Vakzine enthaltend trunkiertes HBV core protein plus Saponin-basierendes Adjuvants**

(71) Anmelder: Rhein Biotech Gesellschaft für neue biotechnologische Prozesse und Produkte mbH, 40595 Düsseldorf (DE)
(72) Erfinder: Melber, Karl, 40591 Düsseldorf (DE); Buchmann, Pascale, 51061 Köln (DE); Janowicz, Zbigniew, 40699 Erkrath (DE)
(74) Vertreter: Hakvoort, Ansgar

(57) **Zusammenfassung**

Die vorliegende Erfindung betrifft eine Zusammensetzung, beinhaltend:
i) ein HBcAg₁₋ₓ, wobei x eine ganze Zahl aus dem Bereich von 100 bis 160 ist, ein Fragment aus diesem Antigen, eine Variante dieses Antigens oder des Fragmentes dieses Antigens oder mindestens zwei davon
ii) ein Adjuvans beinhaltend ein Saponin,
sowie gegebenenfalls
iii) ein HBsAg, ein Fragment aus diesem Antigen, eine Variante dieses Antigens oder des Fragmentes dieses Antigens oder mindestens zwei davon,

wobei
- das Adjuvans als Saponin 50 bis 90 Gew.-% der gemäß der hierin beschriebenen Testmethode aus *Quillaja Saponaria* Molina-Extrakt erhaltenen Fraktion A und 10 bis 50 Gew.-% der gemäß der hierin beschriebenen Testmethode aus *Quillaja Saponaria* Molina-Extrakt erhaltenen Fraktion C enthält,
wobei die Gesamtmenge aus Saponinen der Fraktion A und Saponinen der Fraktion C 100 Gew.-% beträgt;
- das Adjuvans neben dem Saponin Cholesterin, Phosphatidylcholin sowie Decanoyl-N-methylglucamid enthält.

Die Erfindung betrifft auch ein Verfahren zur Herstellung einer Zusammensetzung, die durch dieses Verfahren erhältliche Zusammensetzung, eine Arzneimittelformulierung, die Verwendung der Zusammensetzung oder der Arzneimittelformulierung zur Behandlung und/oder zur Prävention von HBV-Infektionen und HBV-vermittelten Erkrankungen, die Verwendung der Zusammensetzung oder der Arzneimittelformulierung zur Herstellung eines Arzneimittels zur Behandlung und/oder zur Prävention von HBV-Infektionen und HBV-vermittelten Erkrankungen sowie ein Verfahren zur Behandlung und/oder zur Prävention von HBV-Infektionen und HBV-vermittelten Erkrankungen.

## Beschreibung

Die vorliegende Erfindung betrifft eine Zusammensetzung, ein Verfahren zur Herstellung einer Zusammensetzung, die durch dieses Verfahren erhältliche Zusammensetzung, eine Arzneimittelformulierung, die Verwendung der Zusammensetzung oder der Arzneimittelformulierung zur Therapie und/oder zur Prophylaxe von Hepatitis B Virus (HBV)-Infektionen und HBV-vermittelten Erkrankungen, die Verwendung der Zusammensetzung oder der Arzneimittelformulierung zur Herstellung eines Arzneimittels zur Therapie und/oder zur Prophylaxe von HBV-Infektionen und HBV-vermittelten Erkrankungen sowie ein Verfahren zur Therapie und/oder zur Prophylaxe von HBV-Infektionen und HBV-vermittelten Erkrankungen.

Nach Schätzung der Weltgesundheitsorganisation WHO sind oder waren mehr als 2 Milliarden der heute lebenden Menschen mit dem Hepatitis B Virus (HBV) infiziert. Damit gehört HBV zu den bedeutendsten humanpathogenen Erregern mit enormen Auswirkungen auf die menschliche Gesundheit.

Infektionen mit HBV erfolgen über das Blut, besonders von der infizierten Mutter auf das Neugeborene während oder kurz nach der Geburt, durch kontaminierte Blutprodukte und beim Geschlechtsverkehr. HBV stellt ein hepatotropes Virus dar, das sowohl akute wie chronische Infektionen verursachen kann. Während eine Infektion bei der Geburt bzw. in den ersten Lebensmonaten in 90 % der Fälle zu einer chronischen Infektion führt, ist dies bei Infektionen im Erwachsenenalter nur in ca. 10 % der Fälle zu beobachten. In ca. 2 % der infizierten Erwachsenen kann es zu einer fulminanten Hepatitis (akutes Leberversagen) kommen, die mit einer hohen Sterblichkeitsrate verbunden ist.

Eine chronische Infektion ist zu Beginn ohne klinische Symptome, jedoch kann sich nach einer Latenzperiode von 10 bis 30 Jahren durch das persistierende HBV und die pathologische Wechselwirkung des Immunsystems mit den infizierten Leberzellen eine akute Lebererkrankung (aktive, chronische Hepatitis B) entwickeln. Der Anteil von chronischen HBV-Trägern, die eine chronische Hepatitis B entwickeln, liegt bei etwa 25 %. Chronische Hepatitis B kann letztendlich zur Leberzirrhose und/oder primärem Leberzellkarzinom führen. Beide Erkrankungen sind mit einer hohen Sterberate verbunden. Es wird geschätzt, dass es weltweit gegenwärtig ca. 350 Millionen chronisch infizierte HBV-Träger gibt. Jedes Jahr sterben ungefähr eine Million Menschen an den Folgen einer chronsichen HBV-Infektion.

Glücklicherweise kann HBV-Infektionen durch prophylaktische Hepatitis B Impfstoffe vorgebeugt werden. Solche Impfstoffe auf der Grundlage von gereinigtem Hepatitis B Oberflächenantigen (HBsAg) aus dem Plasma chronischer Träger waren erstmals Anfang der Achtziger Jahre verfügbar. Später wurden diese durch rekombinant hergestelltes HBsAg ersetzt. Da jedoch gegenwärtig nicht in allen Ländern die Impfung routinemäßig eingeführt ist, wird HBV auch weiterhin eine Bedrohung der menschlichen Gesundheit darstellen. In diesem Zusammenhang ist es bedeutsam, dass chronische HBV-Träger als infektiös gelten und das Virus übertragen können.

Bei der chronischen Hepatitis B handelt es sich um eine sehr dynamische Erkrankung mit einer Vielzahl von Erscheinungsformen. Im Frühstadium der chronisch verlaufenden Infektion spricht man von der immuntoleranten Phase, die durch die Anwesenheit von viralen Markern im Serum (HBV-DNA, HBsAg, HBeAg) sowie das Fehlen von Leberbeschwerden gekennzeichnet ist. Etwa 25 % der HBV-Träger gehen in eine immunoaktive Phase über, im Verlaufe derer die ersten Lebersymptome auftreten und eine Therapie erforderlich wird. Während dieser immunoaktiven Phase nehmen HBV-DNA im Serum in der Regel ab und Serum-Transaminasen erhöhen sich schubweise. Während HBsAg weiter im Serum vorliegt, gibt es einen kleinen Prozentsatz (ca. 1 %) von Patienten, die spontan eine Serumkonversion von HBeAg positiv zu HBeAg negativ durchlaufen. Diese Serumkonversion ist Anzeichen für das Eintreten in das inaktive Trägerstadium, das durch eine nur geringe Virusaktivität charakterisiert ist. In wenigen Fällen wird zusätzlich eine Serokonversion von HBsAg positiv zu anti-HBsAg positiv beobachtet, was als Zeichen für eine ausgeheilte Infektion gilt. Die aktive chronische Hepatitis B ist durch Nekroinflammation und Fibrose des Lebergewebes, die im Verlauf der Zeit zur Zirrhose und dekompensierter Leberkrankheit führen, gekennzeichnet. Als weitere Komplikation entsteht aus der Zirrhose heraus ein primäres Leberzellkarzinom, wobei manche chronischen HBV-Träger Leberkrebs auch ohne Anzeichen von Zirrhose entwickeln können.

Die Behandlungsmöglichkeiten für aktive chronische Hepatitis B sind sehr beschränkt. Gegenwärtig kann eine Behandlung mit Interferon-α (beispielsweise Intron A^{®} oder Roferon A^{®}) oder antiviralen Substanzen (Lamivudine, Adefovir, Entecavir) erfolgen. Interferon-α wird bevorzugt bei HBeAg positiven Patienten eingesetzt, wobei ungefähr 30 % mit einer HBeAg-Serumkonversion reagieren, die mit einem inaktiven Trägerstatus korreliert. In der Folge zeigen wenige Patienten (ca. 3 %) auch eine HBsAg-Serokonversion als Anzeichen einer vollständigen Heilung. Die Behandlung mit Interferon-α ist jedoch mit schwerwiegenden Nebenwirkungen verbunden, die oftmals einen vorzeitigen Abbruch der Therapie notwendig machen. In HBeAg negativen Patienten zeigt Interferon-α geringe Ansprechraten verglichen mit HBeAg positiven Patienten. Als Alternative können hier antivirale Substanzen, die zur Klasse der Nukleosid/Nukleotid-Reverse-Transkriptase-Inhibitoren gehören, eingesetzt werden. Diese antiviralen Substanzen unterdrücken die Replikation des Virus und verhindern als Folge ein Fortschreiten der Lebererkrankung. Die Serumkonversionsraten für HBeAg positive Patienten sind verglichen mit Interferon-α niedriger und liegen nicht höher als die spontan auftretende Remission. Das Ziel der antiviralen Therapie ist daher, die Krankheit durch Verringerung der viralen Aktivität für eine unbegrenzte Zeitspanne aufzuhalten. Der Langzeitgebrauch von antiviralen Substanzen wird jedoch durch das Auftreten von resistenten Virusmutanten gefährdet. Im Falle der am häufigsten eingesetzten Substanz, Lamivudine, wird inzwischen eine Resistenzrate von bis zu 68 % innerhalb von 4 Jahren beobachtet. Das jüngst eingeführte Adefovir scheint Resistenzen mit geringerer Häufigkeit hervorzurufen. Das grundsätzliche Problem der Virusresistenz stellt jedoch eine inhärente Bedrohung für die Langzeitbehandlung dar, selbst wenn die antiviralen Substanzen einmal in Kombination eingesetzt werden sollten. Deshalb besteht ein dringender Bedarf nach zusätzlichen therapeutischen Mitteln zur Behandlung der chronischen Hepatitis B. Insbesondere wünschenswert sind dabei immunomodulatorische Agentien mit geringen Nebenwirkungen und der Perspektive für eine zeitlich begrenzte Therapie für den Patienten.

Eine solche Alternative ist die spezifische Immuntherapie in Form einer therapeutischen Vakzine. Das Konzept einer therapeutischen Vakzine ist es, das Immunsystem eines chronischen Hepatitis B Patienten spezifisch so zu stimulieren, dass eine adäquate Immunantwort gegen das Virus initiiert wird, die letztendlich zur Kontrolle über das Virus führt und dem Fortschreiten der Lebererkrankung Einhalt gebietet. Eine der offensichtlichen Wirkungen, die eine therapeutische Vakzine zeigen muss, ist das Durchbrechen der vorliegenden Immuntoleranz in der Gegenwart von großen Mengen an viralen Antigenen. Dabei soll die Therapie insbesondere virus-spezifische T-Zellantworten hervorrufen, insbesondere zytotoxische T-Lymphozyten (CTL) gegen diverse Antigene des HBV. Verglichen mit der Situation bei der akuten, ausheilenden Infektion sind gerade diese Immunantworten in Patienten mit chronischer Hepatitis B gar nicht oder nur sehr schwach nachweisbar. Starke virus-spezifische CTL-Antworten werden allgemein als maßgeblich für die Kontrolle viraler Infektionen angesehen. Die Entwicklung eines therapeutischen Impfstoffes hat daher die Induktion von starken antiviralen CTL-Antworten zum primären Ziel.

Rekombinante Subunit-Impfstoffe sind in der Regel nicht in der Lage CTL-Antworten hervorzurufen. In Tiermodellen (Maus) lassen sich signifikante CTL-Antworten mit DNA-Vakzinen, insbesondere DNA-Vakzinen basierend auf einem Hepatitis B Core Antigen (HBcAg)-kodierenden Plasmidvektor, erzielen, jedoch zeigen derartige Vakzine insgesamt beim Menschen bislang nur sehr schwache Immunantworten und stimulieren keine potente CTL-Antwort. Wegen dieser Schwierigkeiten werden gegenwärtig sogenannte "Prime-Boost-Vakzine" entwickelt, wobei abwechselnd mit einer DNA-Vakzine (z. B. kodierend für HBsAg) und einem rekombinanten Virusvektor (z. B. modifizierter Vaccinia Ankara Stamm), der z. B. das HBsAg exprimiert, immunisiert wird(siehe McConkey SJ, Schneider J, Mendy M, Cavenaugh JS, Bertoletti A, Whittle H, Hill AVS: "Safety and Immunogenicity of a novel "prime boost" therapeutic immunisation strategy and Immunogenicity of a novel "prime boost" therapeutic immunisation strategy against hepatitis B virus", Meeting abstract: "The molecular biology of hepatitis B viruses", Bergamo, Italien, September 7-10, 2003).

WO-A-01/98333 beschreibt die Verwendung eines HBc-Antigens, bei dem eine oder mehrere der vier Arginin-Wiederholungseinheiten am C-Terminus entfernt, jedoch der C-terminale Cysteinrest beibehalten wurde, unter anderem zur therapeutischen Behandlung von HBV. Die entfernten Arginin-Wiederholungseinheiten können durch Sequenzen ersetzt sein, welche Epitope aus bakteriellen oder viralen Pathogenen, Parasiten, Allergenen oder mit Krebs assoziierten Antigenen repräsentieren, die von T-Helferzellen, B-Zellen oder zytotoxischen T-Lymphozyten erkannt werden.

WO-A-2005/023297 beschreibt eine Zusammensetzung zur Behandlung von HBV-Infektionen und HBV-vermittelten Erkrankungen, die wenigstens zwei Hepatitis B Oberflächenantigene (HBsAg's), Fragmente davon und/oder diese kodierende Nukleinsäuren enthalten, wobei sich die HBsAg's im HBV-Genotyp in der S-Region und/oder der Prä-S1-Region unterscheiden und wobei die Zusammensetzung kein HBcAg oder dieses kodierende Nukleinsäuren enthält.

WO-A-2005/056051 offenbart unter anderem eine rekombinante Polypeptidzusammensetzung zur Behandlung oder Prophylaxe einer Hepatitis B-Infektion, wobei die Zusammensetzung eine Vielzahl von Komponenten, vorzugsweise mindestens drei oder alle vier ausgewählt aus a) einem Core-Polypeptid-Element, welches eine Sequenz der immunogenen Region des HBV-Core-Proteins beinhaltet, b) einem Oberflächenantigen-Polypeptid-Element, welches eine Sequenz der immunogenen Region des HBV-Oberflächenantigen-Proteins (S, M oder L) beinhaltet, c) einem X-Polypeptid-Element, welches eine Sequenz der immunogenen Region des HBV-X-Proteins beinhaltet und d) eines Polymerase-Polypeptid-Elements, welches eine Sequenz der immunogenen Region des HBV-Polymerase-Proteins beinhaltet, wobei mindestens eines der Elemente mindestens 100 Aminosäuren des jeweiligen HBV-Proteins umfasst.

EP-A-1 136 077 offenbart eine Impfstoffzusammensetzung für die nasale Anwendung, umfassend ein HBsAg und gegebenenfalls ein HBcAg, welche unter anderem auch als therapeutische Vakzine eingesetzt werden kann.

Der vorliegenden Erfindung lag die Aufgabe zugrunde, die sich aus dem Stand der Technik ergebenden Nachteile von Impfstoffen zur Behandlung oder Prophylaxe einer Hepatitis B-Infektion beim Menschen zu überwinden.

Insbesondere lag der vorliegenden Erfindung die Aufgabe zugrunde, eine für die Behandlung eines Menschen geeignete Zusammensetzung anzugeben, welche im Vergleich zu den aus dem Stand der Technik bekannten Zusammensetzungen zu stärkeren Immunantworten, insbesondere starken CTL-Antworten gegen das HBcAg, in Patienten mit einer chronischen HBV-Infektion führt. Dabei sollte die Zusammensetzung bei möglichst vielen Patienten mit einer chronischen HBV-Infektion, unabhängig von deren HLA-Typ, zu einem Durchbrechen der immunologischen Toleranz führen.

Dabei soll die erfindungsgemäße Zusammensetzung unabhängig vom HBV-Genotyp des chronischen Patienten ein Durchbrechen der immunologischen Toleranz ermöglichen und damit zur Ausbildung einer das Virus kontrollierenden Immunantwort führen.

Der vorliegenden Erfindung lag weiterhin die Aufgabe zugrunde, ein Verfahren anzugeben, mit dem mit möglichst geringem präparativen Aufwand eine Zusammensetzung mit den vorstehend beschriebenen Vorteilen erhalten werden kann.

Einen Beitrag zur Lösung der eingangs genannten Aufgaben leistet eine Zusammensetzung, beinhaltend:
i) ein HBcAg₁₋ₓ (HBcAg = HBV core-Antigen), wobei x eine ganze Zahl aus dem Bereich von 100 bis 160, vorzugsweise dem Bereich von 120 bis 150, besonders bevorzugt dem Bereich von 140 bis 149, darüber hinaus bevorzugt dem Bereich von 144 bis 146 und am meisten bevorzugt gleich 145 ist, ein Fragment aus diesem Antigen, eine Variante dieses Antigens oder des Fragmentes dieses Antigens oder eine Mischung aus mindestens zwei davon,
ii) ein Adjuvans beinhaltend ein Saponin,
   sowie gegebenenfalls
iii) ein HBsAg, ein Fragment aus diesem Antigen, eine Variante dieses Antigens oder des Fragmentes dieses Antigens oder eine Mischung aus mindestens zwei davon,
wobei
- das Adjuvans als Saponin 50 bis 90 Gew.-%, besonders bevorzugt 60 bis 80 Gew.-%, noch mehr bevorzugt 65 bis 75 Gew.-% und am meisten bevorzugt etwa 70 Gew.-% der gemäß der hierin beschriebenen Testmethode aus *Quillaja Saponaria* Molina-Extrakt erhaltenen Fraktion A und 10 bis 50 Gew.-%, besonders bevorzugt 20 bis 40 Gew.-%, noch mehr bevorzugt 25 bis 35 Gew.-% und am meisten bevorzugt etwa 30 Gew.-% der gemäß der hierin beschriebenen Testmethode aus *Quillaja Saponaria* Molina-Extrakt erhaltenen Fraktion C enthält, wobei die Gesamtmenge aus Saponinen der Fraktion A und Saponinen der Fraktion C 100 Gew.-% beträgt;
- das Adjuvans neben dem Saponin Cholesterin, Phosphatidylcholin sowie Decanoyl-N-methylglucamid enthält.

Völlig überraschend, dafür aber nicht minder vorteilhaft, wurde festgestellt, dass sich mit der vorstehend beschriebenen, für einen Einsatz beim Menschen grundsätzlich geeigneten Zusammensetzung starke, core-spezifische CTL-Antworten im Tiermodell erhalten lassen, welche in etwa vergleichbar waren mit denjenigen CTL-Antworten, die bei der Verwendung eines HBcAg-kodierenden Plasmidvektors (DNA-Immunisierung) im gleichen Tiermodell erhalten wurden.

Der in den folgenden Ausführungen verwendete Begriff "Polypeptid" betrifft ein Polymer aus Aminosäuren und ist nicht auf eine bestimmte Mindestzahl an Aminosäuren beschränkt. Er umfasst daher ebenso Peptide, Oligopeptide, Dimere, Trimere, Oligomere, Partikel und dergleichen. Weiterhin umfasst der Begriff "Polypeptide" nicht nur die reinen Polymere aus Aminosäuren, welche unmittelbar nach der Translation in den Ribosomen erhalten werden, sondern auch diejenigen Polypeptide, die posttranslational durch Glykosilierung, Acetylierung, Phosphorylierung und dergleichen erhalten werden.

Unter einem "HBcAg₁₋ₓ" wird erfindungsgemäß ein Polypeptid verstanden, welches die Aminosäuren 1 bis x des natürlichen HBcAg's umfasst, vorzugsweise aus den Aminosäuren 1 bis x des natürlichen HBcAg's besteht, wobei unter der Bezeichnung "HBcAg₁₋ₓ" alle denkbaren Polypeptide mit den Aminosäuren 1 bis x (wobei die erste Aminosäure (= Aminosäure 1) diejenige am N-terminalen Ende des Polypeptides ist) des HBcAg's der zur Zeit bekannten 8 HBV-Standardgenotypen A, B, C, D, E, F, G und H zu verstehen sind. Diese 8 Standardgenotypen unterscheiden sich in ca. 8 % ihrer Nukleotidsequenz voneinander (siehe Bartholomeusz, Rev. Med. Virol. 13 (2003), 1-14), wobei die Variation vor allem das HBsAg-Gen betreffen. Vorzugsweise weist der HBV Genotyp A die Referenz-Nukleinsäuresequenz Genbank X02763 bzw. der HBV Genotyp A_{afr} die Referenz-Nukleinsäuresequenz gemäß Genbank AF297621 auf. Für den HBV Genotyp Bₐ ist die Referenz-Nukleinsäuresequenz Genbank D00330, für den Genotyp Bⱼ die Referenz-Nukleinsäuresequenz AB073858. Für den HBV Genotyp C ist die Referenz-Nukleinsäuresequenz Genbank AY206389, für den Genotyp Cₐᵤₛ die Referenz-Nukleinsäuresequenz gemäß Genbank AB048704. Für den Genotyp D ist die Referenz-Nukleinsäuresequenz Genbank X02496, für den Genotyp E Genbank X75657, für den Genotyp F Genbank X69798, für den Genotyp G Genbank AF160501 und für den Genotyp H Genbank AY090454.

Unter der Bezeichnung "Fragment" eines HBcAg₁₋ₓ's wird vorzugsweise ein Polypeptid verstanden, bei dem ein Abschnitt von mindestens 25, vorzugsweise mindestens 50 und besonders bevorzugt mindestens 100 Aminosäuren identisch ist mit einem entsprechenden Abschnitt des HBcAg₁₋ₓ's.

Unter der Bezeichnung "Variante" des HBcAg₁₋ₓ's oder des Fragmentes des HBcAg₁₋ₓ's wird vorzugsweise ein Polypeptid verstanden, bei dem höchstens 10, vorzugsweise höchstens 5 Aminosäuren, besonders bevorzugt höchstens 2 Aminosäuren und am meisten bevorzugt höchstens 1 Aminosäure des HBcAg₁₋ₓ's oder des Fragmentes des HBcAg₁₋ₓ's deletiert, insertiert, substituiert oder aber an das C- und/oder N-terminale Ende angefügt, vorzugsweise substituiert sind bzw. ist. Die Bezeichnung "Variante" umfasst auch Fusionspolypeptide, insbesondere Fusionen mit HBsAg, welche HBcAg₁₋ₓ enthalten.

Unter dem Begriff "Adjuvans" wird erfindungsgemäß eine Verbindung verstanden, welche die Induktion einer Immunantwort, vorzugsweise die Induktion einer CTL-Antwort auf das Antigen ermöglicht.

Die in der erfindungsgemäßen Zusammensetzung enthaltenen, gegenüber den natürlichen HBcAg's trunkierten HBcAg₁₋ₓ's, denen zumindest ein Teil der C-terminalen Nukleinsäurebindungsregion, vorzugsweise die gesamte C-terminale Nukleinsäurebindungsregion fehlt, können durch dem Fachmann bekannte Rekombinationsverfahren erhalten werden.

Eine Möglichkeit besteht darin, das core- bzw. pre-core-Leseraster mittels Polymerasekettenreaktion (PCR) aus einem Virusisolat zu amplifizieren. Denkbar ist auch, die entsprechende DNA-Sequenz auf synthetischem Weg herzustellen und anschließend mittels PCR zu amplifizieren. Das erhaltene PCR-Fragment wird dann in einen Plasmidvektor kloniert. Mittels weiterer PCR-Klonierungen kann dann die entsprechend verkürzte Form in einen Expressionsvektor, beispielsweise in einen Expressionsvektor für E. coli, insertiert werden. Dabei erfolgt die Insertion vorzugsweise dergestalt, dass das für das HBcAg₁₋ₓ bzw. für das Fragment oder die Variante kodierende Gen unter der Kontrolle eines aktiven Promoters steht. Der Expressionsvektor kann gleichzeitig Induktionselemente zur Erhöhung der Expressionsrate enthalten. Nach der Transformation dieses Expressionsvektors in E. coli werden Einzelklone erhalten, welche das HBcAg₁₋ₓ bzw. das Fragment oder die Variante exprimieren. Um die als Antigene dienenden Polypeptide zu erhalten, wird ein Einzelklon in Kulturmedium angeimpft und über Nacht bei 37°C im Schüttelkolben kultiviert. Die E. coli-Kultur wird dann zunächst durch Zentrifugation abgeerntet. Das Bakterienpellet wird in Pufferlösung resuspendiert und anschließend entweder durch Ultraschallbehandlung oder durch Hochdruckhomogenisation aufgeschlossen. Das im Homogenisat befindliche HBcAg bzw. HBcAg-Variante wird durch Fällung mit Ammoniumsulfat konzentriert und anschließend durch Gelfiltration von bakteriellen Wirtskomponenten gereinigt. Partikelbildende HBcAg-Varianten können weiter über eine Geschwindigkeits-Zonenzentrifugation aufgereinigt werden. Partikeldefiziente HBcAg₁₋ₓ's werden idealerweise mit einem N-terminalen His-Tag-Affinitätsmarker konstruiert und mittels einer Ni-NTA-Affinitätschromatographie und anschließender Gelfiltration aufgereinigt. Die so hergestellten HBcAg₁₋ₓ's werden steril filtriert und als solche für die Formulierung der erfindungsgemäßen Zusammensetzung eingesetzt.

Grundsätzlich können die in der erfindungsgemäßen Zusammensetzung enthaltenen HBcAg₁₋ₓ's bzw. deren Fragmente oder Varianten auch auf synthetischem Weg erhalten werden.

Weitere Hinweise zu den Techniken der rekombinanten oder synthetischen Herstellung von Polypeptiden können
- Sambrook, "Molecular Cloning: A Laboratory Manual", Zweite Auflage (1989);
- "DNA Cloning", Volumes I Und II (D. N. Glover, Herausgeber., 1985);
- "Oligonucleotide Synthesis" (M. J. Gait, Herausgeber, 1986);
- "Nucleic Acid Hybridization" (B. D. Hames & S. J. Higgins, Herausgeber, 1984);
- "Transcription and Translation" (B. D. Hames & S. J. Higgins, Herausgeber, 1984);
- "Animal Cell Culture" (R. I. Freshney, Herausgeber, 1986);
- "Immobilized Cells and Enzymes" (IRL Press, 1986);
- B. Perbal, "A Practical Guide to Molecular Cloning" (1984);
- "The Methods in Enzymology series" (Academic Press, Inc.), insbesondere Volumes 154 und 155;
- "Gene Transfer Vectors for Mammalian Cells" (J. H. Miller und M. P. Calos, Herausgeber, 1987, Cold Spring Harbor Laboratory);
- Mayer und Walker, Herausgeber, (1987), "Immunochemical Methods in Cell and Molecular Biology" (Academic Press, London);
- Scopes, (1987), "Protein Purification: Principles and Practice", Zweite Auflage (Springer-Verlag, N.Y.) und
- "Handbook of Experimental Immunology", Volumes I-IV (D. M. Weir und C. C. Blackwell, Herausgeber, 1986)
entnommen werden.

Grundsätzlich können die in der erfindungsgemäßen Zusammensetzung enthaltenen HBcAg₁₋ₓ's partikelbildend oder nicht partikelbildend sein, wobei partikelbildende HBcAg₁₋ₓ'S bevorzugt sind. Partikelbildende HBcAg₁₋ₓ's sind in der Lage, zu Kapsiden mit einem Durchmesser von ca. 27 nm zu assoziieren. Dabei lagern sich 180 bzw. 240 Dimere zu makromolekularen Strukturen zusammen. Gemäß einer besonders bevorzugten Ausführungsform der erfindungsgemäßen Zusammensetzung liegen die HBcAg₁₋ₓ's daher in Form der vorstehend beschriebenen Partikel vor.

Erfindungsgemäß bevorzugte Antigene sind insbesondere das HBcAg₁₋₁₂₄, das HBcAg₁₋₁₂₇, das HBcAg₁₋₁₄₄, das HBcAg₁₋₁₄₅, das HBcAg₁₋₁₄₆, das HBcAg₁₋₁₄₇, das HBcAg₁₋₁₄₈, das HBcAg₁₋₁₄₉, (also Polypeptide, welche die ersten 127, 144, 145, 146, 147, 148 bzw. 149 Aminosäuren des natürlichen HBcAg's umfassen, vorzugsweise aus diesen bestehen) und HBcAg₈₁₋₁₃₃ (ein Fragment des HBcAg's, welches die Aminosäuren 81 bis 133 des natürlich vorkommenden HBcAg's umfasst, vorzugsweise aus diesen besteht). Ein weiterhin bevorzugtes Antigen ist HBcAg_{1-144+AS}, ein Polypeptid, welches die ersten 144 Aminosäuren des natürlich vorkommenden HBcAg's umfasst und welches als 145. Aminosäure eine von der im natürlich vorkommenden HBcAg als 145. Aminosäure auftretenden Glutaminsäure verschiedene Aminosäure AS aufweist, wobei es sich bei dieser von Glutaminsäure verschiedenen Aminosäure AS vorzugsweise um Isoleucin handelt. In der erfindungsgemäßen Zusammensetzung besonders bevorzugt enthaltene Antigene sind HBcAg₁₋₁₄₅ und HBcAg_{1-144+I}.

Weiterhin ist es erfindungsgemäß bevorzugt, dass das HBcAg₁₋ₓ keine HBcAgfremden Epitope, insbesondere keine HBsAg-Epitope aufweist. Am meisten bevorzugt weisen die HBcAg₁₋ₓ's keine Prä-S1-Epitope, insbesondere nicht die Aminosäuren 3 bis 55 des Prä-S1-Antigens, auf. Auch ist es gemäß einer besonderen Ausführungsform der erfindungsgemäßen Zusammensetzung bevorzugt, dass das HBcAg₁₋ₓ, sofern x nicht gleich 48, 61 bzw. 107 ist, an der C-terminalen Position x keinen Cystein-Rest aufweist.

Gemäß einer besonderen Ausführungsform der erfindungsgemäßen Zusammensetzung beinhaltet diese mindestens zwei HBcAg₁₋ₓ's oder Fragmente oder Varianten davon, wobei sich die HBcAg₁₋ₓ's im HBV-Genotyp unterscheiden.

Die erfindungsgemäße Zusammensetzung umfasst ein Adjuvans beinhaltend ein Saponin, wobei das Adjuvans als Saponine vorzugsweise ausschließlich die aus *Quillaja Saponaria* Molina-Extrakt erhaltenen Fraktionen A und C enthält und im Wesentlichen frei von Saponinen der Fraktion B ist.

In diesem Zusammenhang ist es besonders bevorzugt, dass das Adjuvans in Form von Partikeln mit einem mittleren Partikeldurchmesser in einem Bereich von 20 bis 200 nm, besonders bevorzugt 30 bis 100 nm und am meisten bevorzugt 35 bis 45 nm vorliegt. In diesem Zusammenhang ist es weiterhin bevorzugt, dass die einzelnen Adjuvans-Partikel sowohl Saponine der Fraktion A als auch Saponine der Fraktion C beinhalten, die Saponine der Fraktion A und der Fraktion C mithin im Wesentlichen homogen über alle Adjuvans-Partikel verteilt sind.

Die erfindungsgemäß besonders bevorzugten Adjuvantien sind vorzugsweise Saponinkomplexe erhältlich durch ein Verfahren umfassend die folgenden Verfahrensschritte:
IIa) Bereitstellung von PBS-Lösung L₁ mit einem pH-Wert in einem Bereich von 7,2 bis 7,6, besonders bevorzugt 7,3 bis 7,5 und am meisten bevorzugt mit einem pH-Wert von etwa 7,4;
IIb) Bereitstellung einer Lösung L₂ aus 5 bis 15 mg/ml, besonders bevorzugt 7,5 bis 12,5 mg/ml und am meisten bevorzugt etwa 10 mg/ml Cholesterin und 5 bis 15 mg/ml, besonders bevorzugt 7,5 bis 12,5 mg/ml und am meisten bevorzugt etwa 10 mg/ml Phosphatidylcholin in 10 bis 30 gew.-%iger, besonders bevorzugt 15 bis 25 gew.-%iger und am meisten bevorzugt etwa 20 gew.-%iger wässriger Decanoyl-N-methylglucamid-Lösung;
IIc) Bereitstellung einer PBS-Lösung L₃ beinhaltend 50 bis 150 mg/ml, vorzugsweise 75 bis 125 mg/ml und am meisten bevorzugt etwa 100 mg/ml der Mischung des Saponins aus den Fraktionen A und C mit einem pH-Wert in einem Bereich von 6,0 bis 6,4;
IId) Zugabe der Lösung L₂ zur Lösung L₁ im Volumenverhältnis L₂ : L₁ von 1 : 5 bis 1 : 15, vorzugsweise 1 : 7 bis 1 : 13 und am meisten bevorzugt etwa 1 : 10 und der Lösung L₃ zur Lösung L₁ im Volumenverhältnis L₃ : L₁ von 1 : 10 bis 1 : 30, vorzugsweise 1 : 15 bis 1 : 25 und am meisten bevorzugt etwa 1 : 20 und vermischen der so erhaltenen Lösung L₄, wobei dieses Mischen vorzugsweise bei einer Temperatur in einem Bereich von 4 bis 30°C, besonders bevorzugt 15 bis 25°C und am meisten bevorzugt bei Raumtemperatur und über einen Zeitraum von 2 bis 72 Stunden, besonders bevorzugt 12 bis 48 Stunden und am meisten bevorzugt 20 bis 25 Stunden erfolgt;
IIe) Dialysieren der Lösung L₄ gegen PBS-Lösung mit einem pH-Wert in einem Bereich von 7,0 bis 7,8, besonders bevorzugt 7,2 bis 6,6 und am meisten bevorzugt mit einem pH-Wert von etwa 7,4, wobei dieses Dialysieren vorzugsweise bei einer Temperatur in einem Bereich von 4 bis 30°C, besonders bevorzugt 15 bis 25°C und am meisten bevorzugt bei Raumtemperatur und über einen Zeitraum von 0,5 bis 24 Stunden, besonders bevorzugt 1 bis 12 Stunden und am meisten bevorzugt 2 bis 6 Stunden erfolgt;
IIf) Dialysieren der Lösung L₄ gegen PBS-Lösung mit einem pH-Wert in einem Bereich von 6,0 bis 6,4, besonders bevorzugt 6,1 bis 6,3 und am meisten bevorzugt mit einem pH-Wert von etwa 6,2, wobei dieses Dialysieren vorzugsweise bei einer Temperatur in einem Bereich von 4 bis 30°C, besonders bevorzugt 15 bis 25°C und am meisten bevorzugt bei Raumtemperatur und über einen Zeitraum von 0,5 bis 24 Stunden, besonders bevorzugt 1 bis 12 Stunden und am meisten bevorzugt 2 bis 6 Stunden erfolgt;
IIg) gegebenenfalls nochmaliges Dialysieren der Lösung L₄ gegen PBS-Lösung mit einem pH-Wert in einem Bereich von 6,0 bis 6,4, besonders bevorzugt 6,1 bis 6,3 und am meisten bevorzugt mit einem pH-Wert von etwa 6,2, wobei dieses Dialysieren vorzugsweise bei einer Temperatur in einem Bereich von 0 bis 10°C, besonders bevorzugt 2 bis 6°C und am meisten bevorzugt bei 4°C erfolgt.

Durch diese Vorgehensweise werden Adjuvans-Partikel erhalten, die jeweils sowohl Saponine der Fraktion A als auch Saponine der Fraktion C beinhalten.

Erfindungsgemäß am meisten bevorzugte Adjuvantien sind die unter der Handelsbezeichnung ISCOMATRIX^{®} von der Firma CSL Limited, Parkville, Australien erhältlichen Produkte.

Gemäß einer besonderen Ausführungsform der erfindungsgemäßen Zusammensetzung kann diese neben dem Saponinkomplex auch weitere, kein Saponin beinhaltende Adjuvantien als Co-Adjuvantien beinhalten, wobei diese Co-Adjuvantien getrennt von dem Komplex vorliegen oder aber in diesen Komplex eingebunden sein können. Als Co-Adjuvans kann jedes dem Fachmann bekannte Adjuvans eingesetzt werden, welches üblicherweise in Vakzinen eingesetzt wird. Bevorzugte Adjuvantien sind ausgewählt aus der Gruppe umfassend partikuläre Adjuvantien wie beispielsweise Submikro-Öl-in-Wasser-Emulsion, zu denen etwa MF59 (5 % Squalen, 0,5 % Tween^{®} 80 und 0,5 % Span^{®} 85), SAF (10 % Squalen, 0,4 % Tween^{®} 80, 5 % Pluron-blockiertes Polymer sowie thr-Muramyldipeptid (thr-MDP) gehören, Mineralverbindungen wie etwa Aluminiumhydroxid, Aluminiumsulfat oder Aluminiumphosphat, unvollständiges Freundsches Adjuvans, Lipopolysaccharide, N-Acetylglucosaminyl-N-acetylmuramyl-L-alanyl-Dipeptid (GMDP) oder Muramyldipeptid (MDP), GAL-Ceramid, Dimethyldioctadecylammoniumbromid (DDAB), Liposomen, vorzugsweise unfassend Phospholipid und Cholesterin, Mikropartikel aus biologisch abbaubaren Polymeren wie Poly-Lactid-co-glykolid (PLGA), Oligodesoxyribonukleotide mit oder ohne CpG-Motiv, N,N-Di-(β-Stearoylethyl)-N,N-dimethylammonium-chlorid (EQ1), Glykopeptide, Bestandteile der Zellwand von Mycobakterien, quaternäre Amine wie beispielsweise Cetylpyridiniumchlorid und Benzalkoniumchlorid, zwitterionische Amine wie CHAPS (3-(3-Cholamidopropyl)-dimethyl-ammonio)-1-propansulfonat), Dextransulfat, Granulozyten-Makrophagen-Kolonien stimulierender Faktor (GM-CSF), Tumornekrosefaktor (TNF), , Blockcopolymere wie beispielsweise P1205 oder Poloxamer 401, Dimyristoylphosphatidylcholin (DMPC), 3β-Hydroxy-5-androsten-17-on (DHEA), Dimyristoylphosphatidylglycerol (DMPG), Desoxycholsäure-Natriumsalz, Imiquimod, DTP-GDP, 7-Allyl-8-Oxoguanosin, Montanide ISA^{®} 51, Montanide ISA^{®} 720, Murametid, Murapalmitin, Dicetylphosphat, Polymethylmethacrylat (PMMA), PEG-Sorbitanfettsäureester wie Polysorbat20 oder 80 (TWEEN^{®} 20 bzw. TWEEN^{®}80), detoxifizierte Mutanten von bakteriellen ADP-ribosylierenden Toxinen wie Cholera-Toxin oder Pertussis-Toxin oder Mischungen aus mindestens zwei dieser Adjuvantien. Als Co-Adjuvantien besonders bevorzugt sind Lipopolysaccharide, Aluminiumhydroxid, Aluminiumphosphat, GAL-Ceramid, Oligodesoxyribonukleotide mit CpG-Motiv, sowie PEG-Sorbitanfettsäureester.

Eine Übersicht über als Co-Adjuvantien geeignete Verbindungen ist in Cox, J.C. und Coulter, A. R., "Adjuvants-a classification and review of their modes of action" in Vaccine 15:248-256 (Febr. 1997) dargestellt. Die Offenbarung dieser Druckschrift hinsichtlich möglicher Adjuvantien in Impfstoffzusammensetzungen wird hiermit als Referenz eingeführt und bildet einen Teil der Offenbarung der vorliegenden Erfindung.

Gemäß einer weiteren besonderen Ausführungsform der erfindungsgemäßen Zusammensetzung enthält diese neben dem Antigen HBcAg₁₋ₓ und dem Adjuvans noch mindestens ein HBsAg, ein Fragment aus diesem Antigen, eine Variante dieses Antigens oder des Fragmentes dieses Antigens oder eine Mischung aus mindestens zwei davon. In diesem Zusammenhang ist es besonders bevorzugt, wenn nicht nur das HBcAg₁₋ₓ sondern auch das HBsAg in der erfindungsgemäßen Zusammensetzung in partikulärer Form vorliegt.

Bei dem HBsAg handelt es sich um ein 226-Aminosäuren Protein (p24/gp27 oder S-Protein), das in 20-30 nm Lipoproteinpartikel selbstassembliert, in denen ungefähr 100-150 Untereinheiten durch multiple inter-und intra-molekulare Disulfidbindungen quervernetzt sind. Die in der erfindungsgemäßen Zusammensetzung gegebenenfalls enthaltenen HBsAg's können dabei, ebenso wie die HBcAg₁₋ₓ's, von allen zur Zeit bekannten 8 HBV-Standardgenotypen A, B, C, D, E, F, G und H abgeleitet sein. Der Begriff "HBsAg" umfasst weiterhin neben dem 226-Aminosäuren Protein auch die beiden M- und L-Proteine, die neben dem HBsAg noch das 55-Aminosäuren lange Prä-S2-Peptid (M-Protein) bzw. das 55-Aminosäuren lange Prä-S2-Peptid und das 120-Aminosäuren lange Prä-S1-Peptid (L-Protein) umfassen.

Unter der Bezeichnung "Fragment" eines HBsAg's wird vorzugsweise ein Polypeptid verstanden, bei dem ein Abschnitt von mindestens 50, vorzugsweise mindestens 150 und besonders bevorzugt mindestens 200 Aminosäuren identisch ist mit einem entsprechenden Abschnitt des HBsAg's.

Unter der Bezeichnung "Variante" des HBsAg's oder des Fragmentes des HBsAg's wird vorzugsweise ein Polypeptid verstanden, bei dem höchstens 5, vorzugsweise höchstens 2 und besonders bevorzugt höchstens 1 Aminosäure des HBsAg's oder des Fragmentes des HBsAg's deletiert, insertiert, an den Enden angefügt oder substituiert, vorzugsweise substituiert ist.

Gemäß einer bevorzugten Ausführungsform der erfindungsgemäßen Zusammensetzung, bei der diese mindestens ein HBsAg enthält, umfasst diese nicht nur ein HBsAg, sondern, wie dies in der WO-A-2005/023297 beschrieben ist, wenigstens zwei HBsAg's oder Fragmente oder Varianten davon, wobei sich die HBsAg's im HBV-Genotyp in der S-Region und/oder der Prä-S1-Region unterscheiden. In diesem Zusammenhang ist es insbesondere bevorzugt, dass die beiden sich im HBV-Genotyp unterscheidenden HBsAg's in Form homologer Partikel vorliegen, wie dies ebenfalls in der WO-A-2005/02397 beschrieben ist. Unter einem "homologen" HBsAg-Partikel wird dabei ein Partikel verstanden, welches ausschließlich aus HBsAg desselben Subtyps / Genotyps zusammengesetzt ist.

Die HBsAg's können entweder aus dem Serum von Patienten mit chronischer Hepatitis B, auf synthetischem Wege oder als rekombinante Polypeptide durch dem Fachmann bekannte Verfahren erhalten werden. In diesem Zusammenhang sei auf die vorstehend beschriebenen Methoden im Zusammenhang mit der Gewinnung der HBcAg₁₋ₓ verwiesen.

Die erfindungsgemäße Zusammensetzung kann, insbesondere dann, wenn sie als Arzneimittelformulierung vorliegt, neben den Komponenten i), ii) und gegebenenfalls iii) auch noch weitere Zusatzstoffe iv), wie Arzneistoffträger oder Hilfsstoffe beinhalten.

Bevorzugte Arzneistoffträger sind insbesondere Wasser, gepuffertes Wasser, vorzugsweise isotonische Salzlösungen wie PBS (*Phosphate Buffered Saline*)*,* Glucose, Dextrose, Mannitol, Lactose, Stärke, Magnesiumstearat, Zellulose, Magnesiumcarbonat, 0,3 % Glycerol, Hyaluronsäure, Ethanol, Polyalkylenglykole wie Polypropylenglykol, Triglyceride oder dergleichen. Die Art des zugesetzten Arzneistoffträgers hängt insbesondere davon ab, ob die erfindungsgemäße Zusammensetzung für eine orale, eine intranasale, eine intradermale, eine subkutane, eine intramuskuläre oder eine intravenöse Verabreichung formuliert wird. Als Hilfsstoffe können Tonizitätsregler, Benetzungsmittel, Emulgierhilfen oder Puffersubstanzen in der erfindungsgemäßen Zusammensetzung enthalten sein.

Das relative Mengenverhältnis zwischen der Antigenkomponente i) bzw. den Antigenkomponenten i) und iii) zum Adjuvans ii) liegt vorzugsweise in einem Bereich von 1 : 20 bis 20 : 1, besonders bevorzugt in einem Bereich von 1 : 10 bis 10 : 1, darüber hinaus bevorzugt in einem Bereich von 1 : 5 bis 5 : 1 und am meisten bevorzugt in einem Bereich von 1 : 2 bis 2 : 1. Das für den jeweiligen Anwendungsfall optimale Gewichtsverhältnis zwischen Adjuvans und Antigen kann der Fachmann durch einfache Vorversuche, bei denen beispielsweise die Stärke der CTL-Antwort in Abhängigkeit von diesem Mengenverhältnis bestimmt wird, ermitteln.

Sofern die erfindungsgemäße Zusammensetzung neben dem HBcAg₁₋ₓ auch ein HBsAg umfasst, so liegt das Gewichtsverhältnis zwischen dem HBeAg₁₋ₓ, und dem HBsAg vorzugsweise in einem Bereich von 1 : 20 bis 20 : 1, besonders bevorzugt in einem Bereich von 1 : 10 bis 10 : 1, darüber hinaus bevorzugt in einem Bereich von 1 : 5 bis 5 : 1 und am meisten bevorzugt in einem Bereich von 1 : 2 bis 2 : 1. Auch hier kann der Fachmann das optimale Gewichtsverhältnis durch einfache Vorversuche ermitteln.

Die Gesamtkonzentration der Komponenten i), ii) und gegebenenfalls iii) in der erfindungsgemäßen Zusammensetzung (also die Gesamtmenge der Komponenten i), ii) und iii) bezogen auf das Volumen der erfindungsgemäßen Zusammensetzung) liegt, sofern die erfindungsgemäße Zusammensetzung nicht als Lyophylisat vorliegt, vorzugsweise in einem Bereich von 0,1 bis 2.000 µg/ml, besonders bevorzugt in einem Bereich von 0,5 bis 1.500 µg/ml, darüber hinaus bevorzugt in einem Bereich von 1 bis 1.000 µg/ml und am meisten bevorzugt in einem Bereich von 10 bis 500 µg/ml.

Gemäß einer Ausführungsform der erfindungsgemäßen Zusammensetzung umfasst diese
i) ein HBcAg₁₋₁₄₅ sowie
ii) den vorstehend beschriebenen Saponinkomplex als Adjuvans.

Gemäß einer anderen Ausführungsform der erfindungsgemäßen Zusammensetzung umfasst diese
i) ein HBcAg_{1-144+I} sowie
ii) den vorstehend beschriebenen Saponinkomplex als Adjuvans.

Gemäß einer weiteren Ausführungsform der erfindungsgemäßen Zusammensetzung umfasst diese
i) ein HBcAg₁₋₁₄₅,
ii) den vorstehend beschriebenen Saponinkomplex als Adjuvans, sowie
iii) ein HBsAg.

Gemäß einer weiteren Ausführungsform der erfindungsgemäßen Zusammensetzung umfasst diese
i) ein HBcAg_{1-144+I},
ii) den vorstehend beschriebenen Saponinkomplex als Adjuvans, sowie
iii) ein HBsAg.

Vorzugsweise ist die erfindungsgemäße Zusammensetzung des weiteren dadurch gekennzeichnet, dass sie in C57BL/6-Mäusen bei einer Immunisierung gemäß dem hierin beschriebenen Immunisierungsverfahren und anschließender, hierin beschriebener Restimulierung mit MGLKFRQL (einem synthetischen Fragment des HBcAg's) eine gemäß der hierin beschriebenen Methode bestimmte HBcAgspezifische CD8⁺-T-Zell-Frequenz in der Milz von mindestens 30, besonders bevorzugt mindestens 50, darüber hinaus bevorzugt mindestens 75, darüber hinaus noch mehr bevorzugt mindestens 100 und am meisten bevorzugt mindestens 300 CD8⁺ IFN_{γ}⁺ T-Zellen pro 10⁵ CD8⁺ T-Zellen aufweist.

Weiter ist die erfindungsgemäße Zusammensetzung vorzugsweise dadurch charakterisiert, dass sie, sofern sie HBsAg umfasst, in C57BL/6-Mäusen bei einer Immunisierung gemäß dem hierin beschriebenen Immunisierungsverfahren potente Antikörperantworten gegen HBsAg induziert. Die Gesamt-anti-HBs-Antikörpertiter, ermittelt mittels des hierin beschriebenen AUSAB^{®}-Assays belaufen sich dabei im Mittel auf mindestens 100 mIU/mL, vorzugsweise mindestens 500 mIU/mL, besonders bevorzugt auf mindestens 1.000 mIU/mL und am meisten bevorzugt auf mindestens 1.500 mIU/mL. Die Bestimmung der anti-HBsspezifischen IgG-Isotyp-Klassen zeigt, dass sowohl IgG1- als auch IgG2b-Titer erhöht werden, was auf die Ausbildung sowohl einer Th1- als auch Th2-Immunantwort hindeutet.

Einen weiteren Beitrag zur Lösung der Eingangs genannten Aufgaben leistet ein Verfahren zur Herstellung einer Zusammensetzung, umfassend die Verfahrensschritte I), II) und IV) und gegebenenfalls III):
I) Bereitstellen eines HBcAg₁₋ₓ's, wobei x eine ganze Zahl aus dem Bereich von 100 bis 160, vorzugsweise dem Bereich von 120 bis 150, besonders bevorzugt dem Bereich von 140 bis 149, darüber hinaus bevorzugt dem Bereich von 144 bis 146 und am meisten bevorzugt gleich 145 ist, eines Fragmentes aus diesem Antigen, einer Variante dieses Antigens oder des Fragmentes dieses Antigens oder einer Mischung aus mindestens zwei davon,
II) Bereitstellen eines Adjuvans beinhaltend ein Saponin,
   wobei
   - das Adjuvans als Saponin 50 bis 90 Gew.-%, besonders bevorzugt 60 bis 80 Gew.-%, noch mehr bevorzugt 65 bis 75 Gew.-% und am meisten bevorzugt etwa 70 Gew.-% der gemäß der hierin beschriebenen Testmethode aus *Quillaja Saponaria* Molina-Extrakt erhaltenen Fraktion A und 10 bis 50 Gew.-%, besonders bevorzugt 20 bis 40 Gew.-%, noch mehr bevorzugt 25 bis 35 Gew.-% und am meisten bevorzugt etwa 30 Gew.-% der gemäß der hierin beschriebenen Testmethode aus *Quillaja Saponaria* Molina-Extrakt erhaltenen Fraktion C enthält, wobei die Gesamtmenge aus Saponinen der Fraktion A und Saponinen der Fraktion C 100 Gew.-% beträgt;
   - das Adjuvans neben dem Saponin Cholesterin, Phosphatidylcholin sowie Decanoyl-N-methylglucamid enthält.
III) Bereitstellen eines HBsAg's, eines Fragmentes aus diesem Antigen, einer Variante dieses Antigens oder des Fragmentes dieses Antigens oder einer Mischung aus mindestens zwei davon, und
IV) In Kontakt bringen des HBcAg₁₋ₓ's, des Adjuvans und gegebenenfalls des HBsAg's.

Als HBeAg₁₋ₓ bzw. als Fragment oder Variante davon, als Adjuvans beinhaltend ein Saponin sowie als HBsAg bzw. als Fragmentes oder Variante davon sind diejenigen Verbindungen bevorzugt, die bereits im Zusammenhang mit der erfindungsgemäßen Zusammensetzung als bevorzugte Komponenten i), ii) und iii) genannt wurden.

Hinsichtlich der Verfahrensschritte I) und III) ist es bevorzugt, dass das Bereitstellen des HBcAg's und des HBsAg's dergestalt erfolgt, dass die aus dem Plasma von Patienten mit chronischer Hepatitis B (nur im Falle des HBsAg's), durch synthetische Verfahren oder durch rekombinante Expression gewonnenen Antigene oder Antigenfragmente in einem geeigneten Arzneistoffträger vorgelegt werden. Bei diesem Arzneistoffträger handelt es sich vorzugsweise um eine Flüssigkeit, besonders bevorzugt um eine wässrige Flüssigkeit, darüber hinaus bevorzugt um eine wässrige Salzlösung wie PBS. Vor dem in Kontakt bringen mit den übrigen Komponenten im Verfahrensschritt IV) können die auf diese Weise erhaltenen wässrigen Antigenlösungen durch dem Fachmann bekannte Verfahren sterilfiltiert werden.

Dabei ist es gemäß einer bevorzugten Ausführungsform des erfindungsgemäßen Verfahrens bevorzugt, dass das Bereitstellen des HBcAg's und gegebenenfalls des HBsAg's in den Verfahrensschritten I) und III) dergestalt erfolgt, dass diese Antigene zunächst in einem Arzneistoffträger, vorzugsweise in einer wässrigen Salzlösung, in einer Konzentration vorgelegt werden, die mindestens doppelt, besonders bevorzugt mindestens dreimal, darüber hinaus bevorzugt mindestens viermal und am meisten bevorzugt mindestens sechsmal so groß ist wie die Konzentration des HBcAg's bzw. des HBsAg's in der letztendlich angestrebten erfindungsgemäßen Zusammensetzung (soll also beispielsweise die Konzentration an HBcAg in der erfindungsgemäßen Zusammensetzung 10 µg/ml betragen, so ist es bei der Herstellung der Zusammensetzung bevorzugt, dass das HBcAg zunächst in einer wässrigen Salzlösung in einer Konzentration von mindestens 20 µg/ml, besonders bevorzugt mindestens 30 µg/ml, darüber hinaus bevorzugt mindestens 40 µg/ml und am meisten bevorzugt mindestens 60 µg/ml bereitgestellt wird). Sollen sowohl HBcAg als auch HBsAg in der erfindungsgemäßen Zusammensetzung enthalten sein, so ist es bevorzugt, diese gemeinsam in ein und derselben Salzlösung in den vorstehend genannten Konzentrationen (mindestens 2fach, mindestens 3fach, mindestens 4fach bzw. mindestens 6fach überkonzentriert) vorzulegen. Weiterhin ist es erfindungsgemäß bevorzugt, dass diese überkonzentrierten Antigenlösungen vor dem in Kontakt bringen mit dem Adjuvans im Verfahrensschritt IV) für eine Zeitdauer von 10 Minuten bis 4 Stunden, besonders bevorzugt von 20 Minuten bis 2 Stunden und darüber hinaus bevorzugt von 30 Minuten bis 1 Stunde bei einer Temperatur in einem Bereich von 30 bis 60°C, besonders bevorzugt 35 bis 55°C und darüber hinaus bevorzugt 37 bis 50°C inkubiert werden. Nach dem in Kontakt bringen des Antigens mit dem Adjuvans ist es weiterhin bevorzugt, dass die so erhaltene Zusammensetzung für einen Zeitraum von 15 Minuten bis 5 Stunden, besonders bevorzugt von 30 Minuten bis 2 Stunden und darüber hinaus bevorzugt von 45 Minuten bis 2 Stunden bei einer Temperatur von 10 bis 40°C, besonders bevorzugt von 15 bis 30°C, darüber hinaus bevorzugt von 20 bis 25°C und am meisten bevorzugt bei Raumtemperatur inkubiert wird.

Das Bereitstellen des Adjuvans im Verfahrensschritt II) umfasst vorzugsweise die folgenden Verfahrensschritte:
IIa) Bereitstellung von PBS-Lösung L₁ mit einem pH-Wert in einem Bereich von 7,2 bis 7,6, besonders bevorzugt 7,3 bis 7,5 und am meisten bevorzugt mit einem pH-Wert von etwa 7,4;
IIb) Bereitstellung einer Lösung L₂ aus 5 bis 15 mg/ml, besonders bevorzugt 7,5 bis 12,5 mg/ml und am meisten bevorzugt etwa 10 mg/ml Cholesterin und 5 bis 15 mg/ml, besonders bevorzugt 7,5 bis 12,5 mg/ml und am meisten bevorzugt etwa 10 mg/ml Phosphatidylcholin in 10 bis 30 gew.-%iger, besonders bevorzugt 15 bis 25 gew.-%iger und am meisten bevorzugt etwa 20 gew.-%iger wässriger Decanoyl-N-methylglucamid-Lösung;
IIc) Bereitstellung einer PBS-Lösung L₃ beinhaltend 50 bis 150 mg/ml, vorzugsweise 75 bis 125 mg/ml und am meisten bevorzugt etwa 100 mg/ml der Mischung des Saponins aus den Fraktionen A und C mit einem pH-Wert in einem Bereich von 6,0 bis 6,4;
IId) Zugabe der Lösung L₂ zur Lösung L₁ im Volumenverhältnis L₂ : L₁ von 1 : 5 bis 1 : 15, vorzugsweise 1 : 7 bis 1 : 13 und am meisten bevorzugt etwa 1:10 und der Lösung L₃ zur Lösung L₁ im Volumenverhältnis L₃ : L₁ von 1 : 10 bis 1 : 30, vorzugsweise 1 : 15 bis 1 : 25 und am meisten bevorzugt etwa 1 : 20 und vermischen der so erhaltenen Lösung L₄, wobei dieses Mischen vorzugsweise bei einer Temperatur in einem Bereich von 4 bis 30°C, besonders bevorzugt 15 bis 25°C und am meisten bevorzugt bei Raumtemperatur und über einen Zeitraum von 2 bis 72 Stunden, besonders bevorzugt 12 bis 48 Stunden und am meisten bevorzugt 20 bis 25 Stunden erfolgt;
IIe) Dialysieren der Lösung L₄ gegen PBS-Lösung mit einem pH-Wert in einem Bereich von 7,0 bis 7,8, besonders bevorzugt 7,2 bis 6,6 und am meisten bevorzugt mit einem pH-Wert von etwa 7,4, wobei dieses Dialysieren vorzugsweise bei einer Temperatur in einem Bereich von 4 bis 30°C, besonders bevorzugt 15 bis 25°C und am meisten bevorzugt bei Raumtemperatur und über einen Zeitraum von 0,5 bis 24 Stunden, besonders bevorzugt 1 bis 12 Stunden und am meisten bevorzugt 2 bis 6 Stunden erfolgt;
IIf) Dialysieren der Lösung L₄ gegen PBS-Lösung mit einem pH-Wert in einem Bereich von 6,0 bis 6,4, besonders bevorzugt 6,1 bis 6,3 und am meisten bevorzugt mit einem pH-Wert von etwa 6,2, wobei dieses Dialysieren vorzugsweise bei einer Temperatur in einem Bereich von 4 bis 30°C, besonders bevorzugt 15 bis 25°C und am meisten bevorzugt bei Raumtemperatur und über einen Zeitraum von 0,5 bis 24 Stunden, besonders bevorzugt 1 bis 12 Stunden und am meisten bevorzugt 2 bis 6 Stunden erfolgt;
IIg) gegebenenfalls nochmaliges Dialysieren der Lösung L₄ gegen PBS-Lösung mit einem pH-Wert in einem Bereich von 6,0 bis 6,4, besonders bevorzugt 6,1 bis 6,3 und am meisten bevorzugt mit einem pH-Wert von etwa 6,2, wobei dieses Dialysieren vorzugsweise bei einer Temperatur in einem Bereich von 0 bis 10°C, besonders bevorzugt 2 bis 6°C und am meisten bevorzugt bei 4°C erfolgt.

Insbesondere dann, wenn als Adjuvans Komplexe aus Saponinen eingesetzt werden (ISCO-Matrix), ist es erfindungsgemäß bevorzugt, dass zunächst die Komplexe in steriler Form hergestellt bzw. von entsprechenden Anbietern kommerziell erworben werden und anschließend der Saponinkomplex mit der sterilisierten, wässrigen HBcAg-Lösung und gegebenenfalls mit der sterilisierten HBsAg-Lösung oder aber mit einer Mischung aus diesen beiden Lösungen in Kontakt gebracht wird. Auf diese Weise kommt es nicht zum Aufbau eines klassischen ISCOM-Komplexes, bei dem die Antigene in das Innere der ISCO-Matrix eingebaut werden (sogenannte ISCOMs).

Die so erhaltenen Zusammensetzungen können anschließend noch mit weiteren Zusatzstoffen, wie etwa weiteren Arzneistoffträgern und Hilfsstoffen vermischt werden, je nach dem, ob die Zusammensetzung oral, intranasal, intradermal, subkutan, intramuskulär oder intravenös verabreicht werden soll. Als Arzneistoffträger und Hilfsstoffe sind wiederum diejenigen Verbindungen bevorzugt, die bereits im Zusammenhang mit der erfindungsgemäßen Zusammensetzung als bevorzugte Arzneistoffträger und bevorzugte Hilfsstoffe genannt wurden.

Sofern wässrige Lösungen erhalten werden, können diese in wässrigem, sterilem Zustand verpackt oder lyophylisiert werden, wobei die lyophylisierte Zusammensetzung vor der Verabreichung mit einer sterilen Lösung kombiniert wird.

Einen weiteren Beitrag zur Lösung der Eingangs genannten Aufgaben liefern die durch das vorstehend beschriebene Verfahren erhältlichen Zusammensetzungen, wobei diese Zusammensetzungen vorzugsweise die gleichen Eigenschaften aufweisen wie die eingangs beschriebene, erfindungsgemäße Zusammensetzung.

Einen Beitrag zur Lösung der Eingangs genannten Aufgaben leistet auch eine Arzneimittelformulierung, umfassend die erfindungsgemäße Zusammensetzung sowie mindestens einen der eingangs genannten Zusatzstoffe iv).

Einen Beitrag zur Lösung der Eingangs genannten Aufgaben leistet auch die Verwendung der erfindungsgemäßen Zusammensetzung oder der erfindungsgemäßen Arzneimittelformulierung zur Therapie und/oder zur Prophylaxe von HBV-Infektionen und HBV-vermittelten Erkrankungen. Vorzugsweise wird die erfindungsgemäße Zusammensetzung oder die erfindungsgemäße Arzneimittelformulierung zur Verabreichung an Säugetiere, insbesondere Menschen, eingesetzt. Gemäß einer bevorzugten Verwendung der erfindungsgemäßen Zusammensetzung oder der erfindungsgemäßen Arzneimittelformulierung dient diese insbesondere zur Therapie und/oder zur Prophylaxe, vorzugsweise der Therapie, akuter und/oder chronischer, vorzugsweise chronischer HBV-Infektionen.

Der Begriff "Prophylaxe" im Zusammenhang mit einer HBV-Infektion beim Menschen umfasst dabei vorzugsweise
- die Behandlung eines Menschen, welcher noch keinen Kontakt mit HBV hatte, mit dem Ziel, zu verhindern, dass eine Infektion mit HBV erfolgt-,
- die Behandlung eines Menschen, welcher bereits Kontakt mit HBV hatte und der sich in der immunotoleranten chronischen Phase befindet, mit dem Ziel, zu verhindern, dass dieser Mensch in die immunoaktive chronische Phase eintritt, sowie
- die Behandlung eines Menschen, welcher bereits Kontakt mit HBV hatte und der sich im inaktiven Trägerstadium befindet, mit dem Ziel, zu verhindern, dass dieser Mensch erneut in die immunoaktive Phase eintritt.

Der Begriff "Therapie" im Zusammenhang mit einer HBV-Infektion beim Menschen umfasst dabei vorzugsweise
- die Behandlung eines Menschen, welcher bereits Kontakt mit HBV hatte und der sich in der akuten Phase der Infektion befindet,
- die Behandlung eines Menschen, welcher bereits Kontakt mit HBV hatte und der sich in der immunoaktiven chronischen Phase befindet.

Besonders bevorzugt wird die erfindungsgemäße Zusammensetzung oder die erfindungsgemäße Arzneimittelformulierung Patienten verabreicht, die bereits mit HBV infiziert sind.

Personen in der akuten Infektionsphase, der immunotoleranten chronischen Phase, der immunoaktiven chronischen Phase oder im inaktiven Trägerstadium können mit der erfindungsgemäßen Zusammensetzung oder der erfindungsgemäßen Arzneimittelformulierung je nach Bedarf separat oder in Kombination mit anderen Therapien behandelt werden. Bei therapeutischen Anwendungen wird die Zusammensetzung bzw. die Arzneimittelformulierung in einer Menge an einen Patienten verabreicht, die ausreicht, um eine wirksame CTL-Antwort gegen HBV-Antigene hervorzurufen und HBV-assoziierte Symptome und/oder Komplikationen heilt oder zumindest teilweise stoppt. Wird die erfindungsgemäße Zusammensetzung bzw. die erfindungsgemäße Arzneimittelformulierung, wie besonders bevorzugt, zur Therapie der chronischen Hepatitis B eingesetzt, so ist das Ziel der Therapie eine Reduzierung oder im Idealfall eine Eliminierung virusinfizierter Zellen. Da Personen aufgrund einer inadäquaten oder fehlenden CTL-Antwort während der akuten Phase ihrer Infektion eine chronische Hepatitis B entwickeln können, ist es wichtig, eine Menge der immunrelevanten viralen Antigene (HBcAg₁₋ₓ und gegebenenfalls HBsAg) in einer erfindungsgemäßen Zusammensetzung bzw. der erfindungsgemäßen Arzneimittelformulierung bereitzustellen, die ausreicht, um eine CTL-Antwort wirksam zu stimulieren. Mengen, mit denen diese Ziele erreicht werden, werden als "therapeutisch wirksame Dosen" definiert. Die für den jeweiligen Anwendungsfall therapeutisch wirksame Dosis hängt beispielsweise von der genauen Zusammensetzung der erfindungsgemäßen Zusammensetzung bzw. der erfindungsgemäßen Arzneimittelformulierung, der Verabreichungsart, dem Stadium und der Schwere der behandelten Krankheit, dem Gewicht und dem allgemeinen Gesundheitszustand des Patienten sowie dem Urteil des verschreibenden Arztes ab, liegt im Allgemeinen jedoch vorzugsweise in einem Bereich von etwa 0,1 bis 2.000 µg (Gesamtmenge aus Adjuvans, HBcAg₁₋ₓ und gegebenenfalls HBsAg) für einen 70 kg schweren Patienten, besonders bevorzugt in einem Bereich von 0,5 bis 1.500 µg, darüber hinaus bevorzugt in einem Bereich von 1 bis 1.000 µg und am meisten bevorzugt in einem Bereich von 10 bis 500 µg, gefolgt von Auffrischungsdosen in einem Bereich von 0,1 bis 2.000 µg über Wochen bis Monate, je nach der CTL-Antwort eines Patienten, die durch Messung der HBV-spezifischen CTL-Aktivität in den peripheren Blutlymphozyten (PBL) des Patienten bestimmt wird.

Bei der Therapie der chronischen Hepatitis B sollte die Verabreichung solange fortgesetzt werden, bis zumindest die klinischen Symptome oder die Laborindikatoren anzeigen, dass die HBV-Infektion eliminiert wurde oder zumindest unter der Kontrolle des Immunsystems ist, gegebenenfalls noch länger.

Die erfindungsgemäße Zusammensetzung bzw. die erfindungsgemäße Arzneimittelformulierung kann grundsätzlich oral, intranasal, intradermal, subkutan, intramuskulär oder intravenös verabreicht werden.

Einen Beitrag zur Lösung der Eingangs genannten Aufgaben leistet weiterhin die Verwendung der erfindungsgemäßen Zusammensetzung zur Herstellung einer Arzneimittelformulierung zur Therapie und/oder zur Prophylaxe von HBV-Infektionen und HBV-vermittelten Erkrankungen.

In diesem Zusammenhang leistet insbesondere die Verwendung der erfindungsgemäßen Zusammensetzung, umfassend
i) das vorstehend beschriebene HBcAg₁₋ₓ,
ii) das vorstehend beschriebene Adjuvans beinhaltend ein Saponin,
   sowie
iii) das vorstehend beschriebene HBsAg,

zur Herstellung einer Arzneimittelformulierung zur Therapie von HBV-Infektionen und HBV-vermittelten Erkrankungen, vorzugsweise zur Therapie von HBV-Infektionen und HBV-vermittelten Erkrankungen, einen Beitrag zur Lösung der eingangs genannten Aufgaben, bei der die HBV-Infektion oder die HBV-vermittelte Erkrankung durch ein HB-Virus verursacht worden sind, dessen Genotyp oder Subtyp, vorzugsweise Genotyp, sich vom Genotyp oder Subtyp, vorzugsweise Genotyp, des in der erfindungsgemäßen Zusammensetzung enthaltenen HBsAg's und/oder HBcAg's, vorzugsweise HBsAg's, unterscheidet. Wurde beispielsweise die HBV-Infektion oder die HBV-vermittelte Erkrankung durch ein HB-Virus des Genotyps A verursacht, so wird zur Therapie dieser Infektion bzw. dieser Krankheit vorzugsweise eine erfindungsgemäße Zusammensetzung eingesetzt, welche neben den Komponenten i) und ii) als Komponente iii) ein HBsAg des Genotyps B, C, D, E, F, G oder Mischungen von HBsAg's dieser Genotypen verwendet, wobei auch ein HBsAg des Genotyps A enthalten sein kann.

Des weiteren leistet ein Verfahren zur Therapie und/oder zur Prophylaxe von HBV-Infektionen und HBV-vermittelten Erkrankungen einen Beitrag zur Lösung der eingangs genannten Aufgaben, bei dem einem noch nicht mit HBV in Kontakt getretenen Menschen oder einem bereits mit HBV in Kontakt getretenen Menschen, vorzugsweise einem HBV-infizierten Menschen, besonders bevorzugt einem an chronischer Hepatitis B leidenden Patienten, eine therapeutisch wirksame Dosis der erfindungsgemäßen Zusammensetzung bzw. der erfindungsgemäßen Arzneimittelformulierung vorzugsweise in der vorstehend beschriebenen Art und Weise verabreicht wird.

Auch hier kann es besonders vorteilhaft sein, dem bereits mit HBV in Kontakt getretenen Menschen, vorzugsweise dem HBV-infizierten Menschen, besonders bevorzugt dem an chronischer Hepatitis B leidenden Patienten, eine solche Zusammensetzung zu verabreichen, welche neben dem HBcAg₁₋ₓ und dem Adjuvans auch ein HBsAg beinhaltet, welches sich von einem HBV-Genotyp oder HBV-Subtyp, vorzugsweise HBV-Genotyp, ableitet, der sich vom Genotyp oder Subtyp, vorzugsweise Genotyp, desjenigen HB-Virus unterscheidet, welcher ursächlich ist für die HBV-Infektion bzw. die HBV-vermittelte Erkrankung des zu behandelnden Patienten.

Die Erfindung wird nun anhand nicht limitierender Figuren und Beispiele näher erläutert.

Die Figur 1 zeigt die HBs₂₀₈₋₂₁₅-spezifische T-Zellantwort HBsAg-transgener Mäuse bei der Verwendung erfindungsgemäßer Zusammensetzungen, welche HBcAg und HBsAg beinhalten, mit ISCOMATRIX® als Adjuvans, jeweils bestimmt nach 12 Tagen nach der 2. Immunisierung.

Die Figur 2 zeigt die die B-Zell-Antwort bei der Verwendung erfindungsgemäßer Zusammensetzungen bei der Immunisierung HBsAg-transgener Mäuse, veranschaulicht durch den AUSAB-anti-HBsAG-Titer, jeweils bestimmt nach 12 Tagen nach der 2. Immunisierung.

Die Figur 3 zeigt die HBc₉₃₋₁₀₀-spezifische T-Zellantwort HBV-transgener Mäuse bei der Verwendung erfindungsgemäßer Zusammensetzungen, welche HBcAg und HBsAg beinhalten, mit ISCOMATRIX^{®} als Adjuvans, jeweils bestimmt nach 14 Tagen nach der 2. Immunisierung.

Die Figur 4 zeigt die HBs₁₉₀₋₁₉₇-spezifische T-Zellantwort HBV-transgener Mäuse bei der Verwendung erfindungsgemäßer Zusammensetzungen, welche HBcAg und HBsAg beinhalten, mit ISCOMATRIX^{®} als Adjuvans, jeweils bestimmt nach 14 Tagen nach der 2. Immunisierung.

Die Figur 5 zeigt den HBsAg-Gehalt im Serum HBV-transgener Mäuse bei der Verwendung erfindungsgemäßer Zusammensetzungen, welche HBcAg und HBsAg beinhalten, mit ISCOMATRIX® als Adjuvans, jeweils bestimmt nach 14 Tagen nach der 2. Immunisierung.

### METHODEN

### Herstellung der Saponine der Fraktion A und C

Die Herstellung der Saponine der Fraktion A und C aus dem Roh-Extrakt von *Quillaja Saponaria* Molina erfolgt, wie im Beispiel 1 der WO-A-96/11711 beschrieben.

### HBsAg-transgene Mäuse

C57B1/6J-TgN(A1b1HBV)44Bri - transgene Mäuse, die das HBsAg des Subtyps ayw (kodiert durch HBV-Sequenz mit der Accession No. V01460 J02203) exprimieren, wurden von Jackson Laboratory (Bar Harbor, ME, USA) bezogen. Männliche und weibliche Tiere im Alter von 8-16 Wochen wurden zur Immunisierung verwendet.

### Immunisierung von HBsAg- trans genen Mäusen

C57B1/6J-TgN(A1b1HBV)44Bri - transgene Mäuse wurden mit den in Tabelle 1 angegebenen Mengen an HBcAg, HBsAg und Adjuvans in jeweils 100 µl PBS immunisiert, wobei jeweils zweimal im Abstand 21 Tagen die jeweils angegebenen Mengen intramuskulär injiziert wurden. Für die DNA Immunisierung wurde das Plasmid pCI/HBsAg_{adw} in Mengen von 2 × 50 µg in jeweils 50 µl in je einen *Tibialis anterior-* Muskel injiziert.

### HBV-transgene Mäuse

Tg(HBV_{ayw}1.3genome)Chi32 (lineage 1.3.32) - transgene Mäuse (beschrieben in Guidotti L, Matzke B, Schaller H, and Chisari FV. High-level hepatitis B virus replication in transgenic mice. J Virol 1995; 69:6158-69) wurden gezüchtet und unter Standard-pathogenfreien Bedingungen gehalten. Männliche und weibliche Tiere im Alter von 8-16 Wochen wurden zur Immunisierung verwendet.

### Immunisierung von HBV(1.3.32) - transgenen Mäusen

HBV(1.3.32) - transgene Mäuse wurden mit den in Tabelle 2 angegebenen Mengen an HBcAg, HBsAg und Adjuvans in jeweils 100 µl PBS immunisiert, wobei jeweils zweimal im Abstand 21 Tagen die jeweils angegebenen Mengen subkutan injiziert wurden. Für die DNA Immunisierung wurde das Plasmid pCI/HBsAg_{adw} in Mengen von 2 × 50 µg in jeweils 50 µl in je einen *Tibialis anterior-* Muskel injiziert.

### Bestimmung der HBsAg- bzw. HBcAg -spezifischen CD8⁺-T-Zell-Frequenzen in der Milz

12-14 Tage nach der zweiten Immunisierung wurden den Tieren die Milzen entnommen und Zellsupensionen aus den Milzzellen hergestellt. Die Herstellung von Milzzellsuspensionen ist in Schirmbeck R., Böhm W., Fissolo N., Melber K., und Reimann J.: "Different imunogenicity of H-2 Kb-restriucted epitopes in natural variants of the hepatitis B surface antigen", Europ. J. Immunonol. 2003, 33: 2429-38 beschrieben. Die Milzzellen wurden für 4 Stunden in RPMI-1640 Medium mit 0,25 µM HBcAg-spezifischem Peptid (MGLKFRQL; Jerini BioTools, Berlin, DE, SEQ ID Nr. 01) bzw. mit 0,25 µM HBsAg-spezifischem Peptid (VWLSVIWM; Jerini BioTools, Berlin, DE, SEQ ID Nr. 02) und jeweils 5 µg/ml Brefeldin A (BFA) (Katalognr. 15870, Sigma) bei 37°C inkubiert (Restimulation). Die restimulierten Zellen wurden geerntet und deren Oberfläche mit anti-CD8 mAb (anti-Maus CD8a-PE-Konjugat; Katalognr. 55303; BD Biosciences, Heidelberg, DE) gefärbt, fixiert, permeabilisiert und eine Färbung auf cytoplasmatisches IFNγ (anti-Maus IFNy-FITC-Konjugat; Katalognr. 554411; BD-Biosciences, Heidelberg, DE) durchgeführt. Die Frequenzen von core- bzw. surface-spezifischen CD8⁺IFN_{γ}⁺CTL's wurden durch FACS Analyse bestimmt. Der Durchschnittswert für CD8⁺IFN_{γ}⁺-T-Zellen/10⁵ CD8⁺T-Zellen ist gezeigt.

### Bestimmung des Antikörper-Isotyps

96-well-Mikrotiterplatten (Nunc, Wiesbaden, DE) wurden mit dem zur Immunisierung eingesetzten HBsAg (2 µg/mL in Carbonatpuffer) über Nacht bei 4°C beschichtet (100 µL pro Vertiefung).

Zu Beginn der ELISA-Durchführung wurden die beschichteten Platten dreimal mit PBST (Phosphat-gepufferte Salzlösung mit 0.5 % Tween 20) gewaschen. Die Vertiefungen wurden mit 0.2 % Gelatine 1 h bei 37°C geblockt. Die zu bestimmenden Mausplasmen/ -seren wurden in 2er-Stufen in PBS verdünnt (im Bereich 1:250 bis 1:32 000) und jeweils 100 µL der Verdünnungen in die entsprechenden Vertiefungen gegeben (in Duplikaten) und 1 h bei 37°C inkubiert. Als Kontrolle diente Serum von nicht immunisierten Mäusen. Anschließend wurde die Mikrotiterplatte fünf Mal mit PBST gewaschen und mit einem Isotyp-spezifischen Zweitantikörper (Peroxidase-konjugiert) inkubiert. Zum Nachweis von IgG1 wurde gamIg1 (Katolognr. GAM/IgG1/PO) und zum Nachweis von IgG2b gamIg2b (Katalognr. IgG2b/PO; Nordic Immunological Laboratories, Tilburg, Niederlande) eingesetzt. Anschließend wurde die Farbreaktion mit o-Phenylenediamin (oPD) (Sigma; Katalognr. P2903) als Substrat (1 mg/ml oPD mit 1 µl/mL 30 % H₂O₂) durchgeführt (100 µL pro Vertiefung). Die Farbreaktion wurde nach 10-15 Minuten mittels 1 N H₂SO₄ (50 µL pro Vertiefung) gestoppt und die optischen Dichte bei 492nm im Spektrophotometer (SpectraMax plus; Molecular Devices, Ismaning, DE) gemessen.

### Bestimmung des Gesamt-anti-HBs- Antikörpertiters

Der anti-HBs-Antikörpertiter in den Seren der immunisierten Mäuse wurde bestimmt im IMX Automated Immunoassay Analyzer (Abbott Diagnostics, Wiesbaden, DE) unter Verwendung des IMX AUSAB^{®} Testkits (Referenznr. 2226-21, Abbott, Wiesbaden, DE). Die Durchführung des Assays erfolgte wie vom Hersteller beschrieben.

### Bestimmung des HBsAg im Serum

Der HBsAg-Gehalt in den Seren der immunisierten Mäuse wurde bestimmt im IMX^{™} Automated Immunoassay Analyzer (Abbott Diagnostics, Wiesbaden, DE) unter Verwendung des IMX HBsAg V2 Reagenz (Katalognr. 222822, Abbott, Wiesbaden, DE). Die Durchführung des Assays erfolgte wie vom Hersteller beschrieben. Da es sich um einen nicht-quantitativen Test handelt, wurden die Adsorptionswerte gegen ein rekombinantes HBsAg bekannter Konzentration abgeglichen. Die HBsAg-Menge wird dementsprechend in willkürlichen Einheiten angegeben.

### Herstellung der Antigene

a) HBcAg₁₋₁₄₅
   Über *Bam*HI-Restriktion wurde aus dem Vektor pBR322 (Bolivar F., Rodriguez R.L., Greene P.J., Betlach M.C., Heyneker H.L., Boyer H.W.: "Construction and Characterization of new cloning vehicles. II. A multipurpose Cloning System", Gene. 1977; 2(2): 95-113) und dem zirkularisierten Hepatitis B Virus Genom (HBV320, 3.182 Basenpaare; Bichko V, Pushko P, Dreilina D, Pumpens P, Gren E (1985), "Subtype ayw variant of hepatitis B virus", FEBS 185: 208-212) das Plasmid pHB320 (7.543 Basenpaare) konstruiert, welches das komplette HBV Genom trägt.
   Nach *Hha*I-Restriktion und S1-Nuklease-Abbau der überhängenden Einzelstrangsequenzen wurde ein 1.000 Basenpaare langes Fragment, das die Gensequenzen für das pre-core/core-Gen (*core open reading frame*) trägt, in den Vektor pBR322 P*trp* (Ikehara M, Ohtsuka E, Tokunaga T et al. (1984) Synthesis of a gene for human growth hormone and its expression in E. coli. Proc. Natl. Acad. Sci. USA 81:5956-5960) über *Blunt-end*-Ligation kloniert. Dieser wurde zuvor *Bam*HI*lSal*I geschnitten und die überstehenden Enden mit DNA-Polymerase aufgefüllt. Das resultierende Plasmid pGC74 (6.046 Basenpaare) trägt den Tryptophan-Promoter und das HBc-Gen in entgegengesetzter Orientierung. Diese Orientierung ermöglicht die nachfolgenden Klonierungsschritte durch die Rekonstitution singulärer Schnittstellen zur Optimierung der HBcAg-Expressionskassette.
   Zum Wechsel der Orientierung des HBc-Fragmentes wurde pGC74 mit *Bam*HI restringiert, die überstehenden Enden mit DNA-Polymerase aufgefüllt und mit *Sal*I nachgeschnitten. Dieses Fragment wurde mit dem Vektor pBR322 P*trp* ligiert. Dieser wurde zuvor mit *Ava*I linearisiert, die Enden mit DNA-Polymerase aufgefüllt und mit *Sal*I nachgeschnitten. Das so konstruierte Plasmid pGC2 (5.552 Basenpaare) trägt den Tryptophan-Promoter und das HBc-Gen in gleicher Orientierung.
   Dieses Plasmid wurde mit *Sal*I geschnitten, die überstehenden Enden mit der Nuklease *Bal*31 entfernt, mit *Eco*RI nachgeschnitten, die dabei entstehenden überhängenden Enden mit DNA-Polymerase aufgefüllt und erneut ligiert. Es resultierte Plasmid pHBc10 (4.825 Basenpaare), mit einer verkürzten Sequenz zwischen Tryptophan-Promoter und HBc-Gen zum Erhalt einer optimalen P*trp*-Shine-Dalgarno-HBc-Struktur bzw. Distanz.
   Zur Optimierung der Genexpression wurde ein 1.044 Basenpaar-*Hha*I-HBc-Fragment aus pHBc10 nach Restriktion mit *Hha*lI mit S1-Nuklease verdaut und in den Vektor pBR322-*trp*-I, ein modifiziertes pBR322-Plasmid, kloniert. Aus dieser Konstruktion resultierte pHBc3 (7.108 Basenpaare), mit der optimierten Expressionssequenz des HBc-Gens unter der Kontrolle des Tryptophan-Promoters.
   Das Plasmid pHBc3 wurde mit Kpn21 geschnitten und in die Schnittstelle an der Aminosäureposition 144 des HBc-Gens ein synthetischer Polylinker *Eco*RV*-Cla*I*-Pvu*I (5'-TCCGGAGATATCGATCGTCCGGA-3', SEQ ID Nr. 03) eingesetzt. Hieraus resultierte Plasmid pHBc1615 (7.129 Basenpaare). pHBc1615 wurde mit *Cla*I linearisiert und ein synthetischer Polylinker mit einem zusätzlichen Isoleucin-Codon an Position 145 sowie einem Terminationscodon eingesetzt. Es wurde Plasmid pHBc2-7 erhalten.
   Zur Konstruktion des HBcAg₁₄₅-Expressionsvektors (p327c145) wurde ein PCR-Fragment des HBcAg-Gens aus Plasmid pHBc3 mit den Enzymen *Eco*RI und *Bam*HI geschnitten und in einen *Eco*RI/*Bam*HI geschnittenen pBR322 - Vektor kloniert. Durch den Verdau von pBR322 mit *Eco*RI/*Bam*HI wird ein 290 bp langes Fragment des Tetracyclin-Resistenzgens eliminiert. Das so resultierende Plasmid pBRC (4.608 bp) trägt als einzigen Resistenzmarker nunmehr eine Ampicillin-Resistenz. Ein zweites PCR-Fragment wurde von der Tryptophan-Genregion des Plasmids pHBc3 generiert, mit den Enzymen *Eco*RI und *Nco*I geschnitten und schließlich mit E-*co*RI/*Nco*I-restringiertem pBRC ligiert.
   Das resultierende Plasmid pCAK (4.755 bp) trägt ein 552 bp HBcAg-Fragment unter Kontrolle des Tryptophan-Promotors und einem Ampicillin-Resistenzgen als Marker. Das Ampicillin-Resistenzgen wiederum wurde ausgetauscht gegen ein Kanamycin-Resistenzgen (als *Ssp*I/*Nru*I*-*Fragment nach Klenow-Füllreaktion), das aus einem kommerziell erhältlichen Plasmid pREP-4 (Qiagen, Hilden, DE) stammte. Das entstandene Plasmid pCAK-NEO5 trägt das 552 bp HBcAg-Fragment unter Kontrolle des Tryptophan-Promotors mit einem Kanamycin-Resistenzmarker. Durch Restriktion mit *Hind*III und *Bam*HI wurde aus pCAK-NEO5 ein Fragment mit dem 552 bp HBcAg sowie dem Kanamycin-Resistenzgen erhalten und in einen ebenfalls *Hind*III/*BAm*HI *-* geschnittenen pBR327-Vektor kloniert. Das so entstandene Plasmid pHBc-I719 (5.038 bp) trägt nun zwei Resistenzmarker (Ampicillin und Kanamycin) sowie ein HBcAg (komplettes Gen kodierend für 183 Aminosäuren) unter Kontrolle des Tryptophan-Promotors.
   Das oben beschriebene Plasmid pHBc2-7 trägt ein C-terminal verkürztes HBcAg-Fragment mit einem zusätzlichen Isoleucin an Aminosäureposition 145 (HBcAg_{1-144+I}). Aus dieser Sequenz wurde ein 375 bp-Fragment durch Restriktion mit *Xba*I/*Hpa*I isoliert. Plasmid pHBc-I719 wurde mit den Enzymen *Xba*I und *Nru*I verdaut um den C-terminalen Teil des HBcAg-Gens sowie einen Teil der Vektorsequenz zu entfernen. Der verbleibende Teil des pHBc-I719 wurde mit dem oben beschriebenen 375 bp- *Xba*I/*Hpa*I*-* Fragment ligiert. Der resultierende Vektor ist pHBC-I721 (4287 bp) mit einem verkürzten HBcAg - Gen (HBcAg_{144+I}) unter Kontrolle des Tryptophan-Promotors. Die C-terminale Aminosäure Isoleucin des HBcAg_{1-144+I} wird mittels PCR Mutagenese des Expressionsvektors pHBC-I721durch Glutaminsäure ersetzt. Der resultierende Expressionsvektor hat die Bezeichnung p327c145 und kodiert für HBcAg₁₄₅.
   Kompetente E. coli Zellen (Stamm BL21: Genotyp B F⁻dcm ompT hsdS (r_{B}⁻ m_{B}⁻)gal) wurden mit Plasmid p327c145 transformiert und nach Plattierung auf LB Agar mit Ampicillin über Nacht bei 37°C erhalten. Mit jeweils einer Einzelkolonie werden 300 ml M9 Minimalmedium (unter Zusatz von 1 % Casaminosäuren, Difco Kat.-Nr. 223050, Becton-Dickinson, Heidelberg) und 0,2 % Glukose beimpft. Die Kultivierung erfolgte über Nacht bei 37°C auf einem Rundschüttler in 11-Erlenmeyerkolben. Die Kulturen erreichten üblicherweise eine optische Dichte (OD₅₄₀ₙₘ) zwischen 2 und 5.
   Die Zellen wurden abzentrifugiert und das Pellet in Lysepuffer (50 mM TRIS/HCl pH 8,0; 5 mM EDTA, 100 µg/ml PMSF, 0,08 % Triton-X-100) resuspendiert. Anschließend wurden die konditionierten Zellen auf Eis durch Ultraschallbehandlung (3 × 15 Sekunden bei 22 kHz) lysiert. Nach Abzentrifugieren der Zelldebris wurden die Proteine aus dem Überstand mit Ammoniumsulfat (33 % Sättigung) für 4-20 Stunden bei 4°C präzipitiert. Das Pellet wurde in PBS mit 0,1 % Triton-X-100 resuspendiert und anschließend jeweils 5-10 ml der erhaltenen Lösung auf eine Sepharose CL4B-Chromatographiesäule (2,5×85 cm) aufgeladen. Die Elution erfolgte mit PBS ohne Zusatz von Triton-X-100. Die Anwesenheit von HBcAg-Polypeptiden in den Einzelfraktionen wurde mittels Polyacrylamidgelelektrophorese / Coomassie-Färbung untersucht. Positive Fraktionen wurden vereinigt und mittels Ammoniumsulfat-Präzipitation (33 % Sättigung) für 20 Stunden bei 4°C konzentriert. Die Proteinpellets wurden in PBS mit einer Konzentration zwischen 3-20 mg/ml resuspendiert und anschließend über Nacht dialysiert gegen 1.000 Volumina des gleichen Puffers. Das HBcAg₁₋₁₄₅ in PBS wurde sterilfiltriert und bei -20°C gelagert.
b) HBsAg
   HBsAg des Genotyps A (Subtyp adw₂), des Genotyps C (Subtyp adr) bzw. des Genotyps D (Subtyp ayw4) wurde hergestellt wie beschrieben in: Brocke P, Schaefer S, Melber K, Jenzelewski V, Müller F, Dahlems U., Bartelsen O., Park KN, Janowicz ZA, Gellissen G: "Recombinant hepatitis B vaccines: disease characterization and vaccine production" (2005) in Gellissen G (Herausgeber): "Production of recombinant proteins - novel microbial and eucaryontic expression systems", Seiten 319-360, Wiley-VCH, Weinheim) und kann von Rhein Biotech, Düsseldorf, Germany, bezogen werden.

### BEISPIEL 1

In einer ersten Versuchsreihe wurde als Adjuvans der unter der Produktbezeichnung ISCOMATRIX® (CSL Limited, Parkville, Australia) kommerziell erhältliche Saponinkomplex eingesetzt. Als Positivkontrolle diente HBsAg_{adw2}-Plasmid-DNA, als Negativkontrolle reines PBS. Die erfindungsgemäße Zusammensetzung beinhaltete PBS, HBc₁₋₁₄₅, HBsAg_{adw2} sowie ISCOMATRIX®. Die Mengen der einzelnen Komponenten sind in der Tabelle 1 angegeben.

Das Plasmid pCI/HBsAg_{adw2} wurde wie in Schirmbeck R, Dikopoulos N, Kwissa M, Leithäuser F, Lamberth K, Buus S, Melber K, and Reimann J (2003): "Breaking tolerance in hepatitis B surface antigen transgenic mice by vaccination with cross-reactive, natural HBsAg variants", Eur. J. Immunol.: 3342-52, beschrieben, hergestellt und mit QIAGEN-tip 500 (Katalognr. 12162; Qiagen, Hilden, Deutschland) wie vom Hersteller beschrieben isoliert.

Zur Formulierung der erfindungsgemäßen Zusammensetzung wurden zunächst unter aseptischen Bedingungen HBsAg_{adw2} und HBcAg₁₋₁₄₅ zusammengegeben, mit PBS auf die 4-fache Konzentration des finalen Ansatzes verdünnt und dann 30 Minuten bei 37°C unter Schütteln inkubiert. Anschließend wurde ISCOMATRIX®, so wie vom Hersteller als wässrige Dispersion geliefert, zugefügt und mit PBS die in der Tabelle 1 angegebene Endkonzentration des Antigens eingestellt. Die so hergestellte Zusammensetzung wurde für eine Stunde bei Raumtemperatur (20-25°C) inkubiert, anschließend über Nacht (12-18 Stunden) bei 2-8°C gelagert und am nächsten Tag zur Immunisierung von HBsAgtransgenen Mäusen eingesetzt.

**Tabelle 1**

| Versuchsgruppe | Zur Immunisierung eingesetzte Zusammensetzung |
|---|---|
| A (n. erf.) | 100 µl PBS |
| B (n. erf.) | 2 × 50 µg HBsAg_{adw2}-Plasmid-DNA in 50 µl PBS |
| C (erf.) | 5 µg ISCOMATRIX^{®}, 4 µg HBcAg₁₋₁₄₅, 10 µg HBsAg_{adw2} in 100 µl PBS |

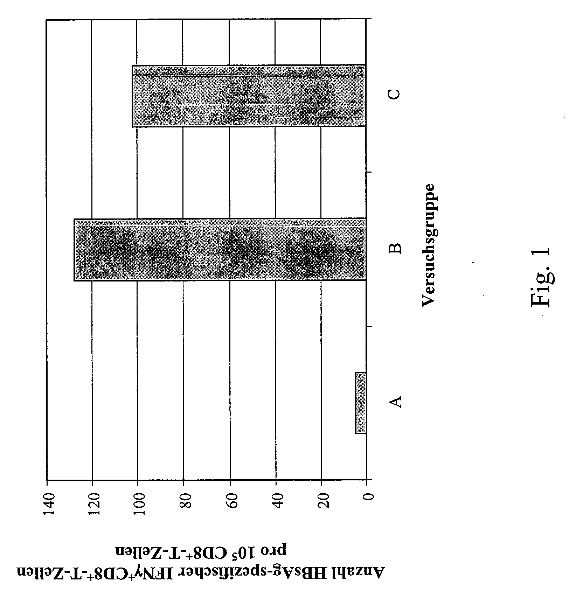

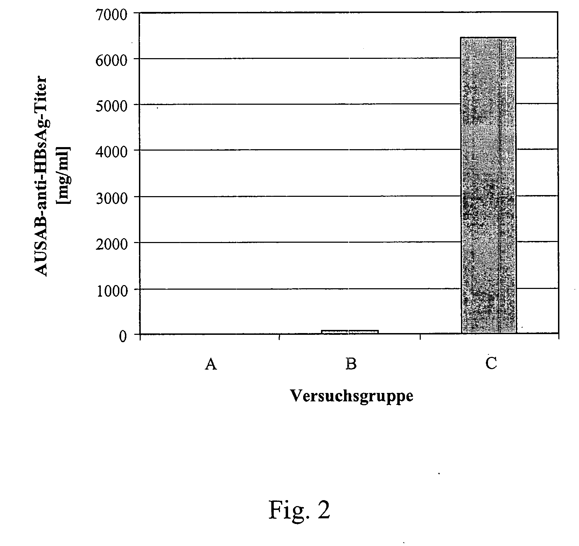

Wie den Ergebnissen in den Figuren 1 und 2 zu entnehmen ist, kann mit der erfindungsgemäßen Zusammensetzung die Immuntoleranz gegen HBsAg in HBsAgtransgenen Mäusen durchbrochen werden. Dieses spricht für die Eignung der erfindungsgemäßen Zusammensetzung, auch bei einer chronischen Hepatitis B-Infektion beim Menschen eine gegen HBV-gerichtete Immunantwort zu induzieren.

### BEISPIEL 2

In einer weiteren Versuchsreihe wurde wiederum als Adjuvans der unter der Produktbezeichnung ISCOMATRIX^{®} kommerziell erhältliche Saponinkomplex eingesetzt. Als Negativkontrolle diente eine Zusammensetzung beinhaltend PBS und ISCOMATRIX^{®}. Die erfindungsgemäße Zusammensetzung beinhaltete PBS, HBc₁₋₁₄₅, ISCOMATRIX^{®} sowie HBsAg_{adr}. Die Mengen der einzelnen Komponenten sind in der Tabelle 2 angegeben.

Die Formulierung der erfindungsgemäßen Zusammensetzungen erfolgte wiederum gemäß der im Zusammenhang mit Beispiel 1 beschriebenen Vorgehensweise, wobei auch hier durch Zugabe von PBS die in der Tabelle 2 angegebenen Endkonzentrationen der Antigene eingestellt wurden. Die so hergestellten Zusammensetzungen wurden für eine Stunde bei Raumtemperatur (20-25°C) inkubiert, anschließend über Nacht (12-18 Stunden) bei 2-8°C gelagert und am nächsten Tag zur Immunisierung von HBV-transgenen Mäusen eingesetzt.

**Tabelle 2**

| Versuchsgruppe | Zur Immunisierung eingesetzte Zusammensetzung |
|---|---|
| D (n. erf.) | 4 µg ISCOMATRIX^{®} in 100 µl PBS |
| E (erf.) | 10 µg HBcAg₁₋₁₄₅, 10 µg HBsAg_{adr}, 4 µg ISCOMATRIX^{®} in 100 µl PBS |

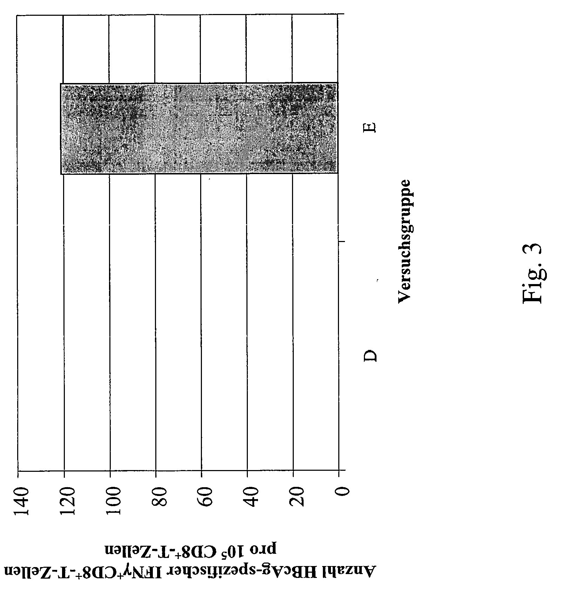

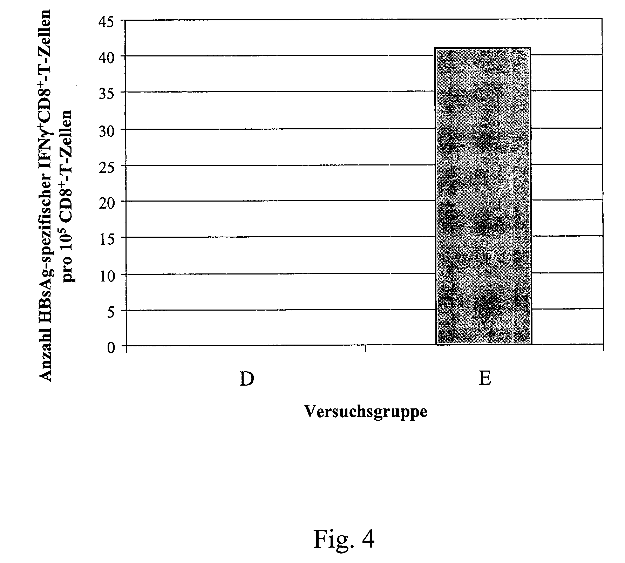

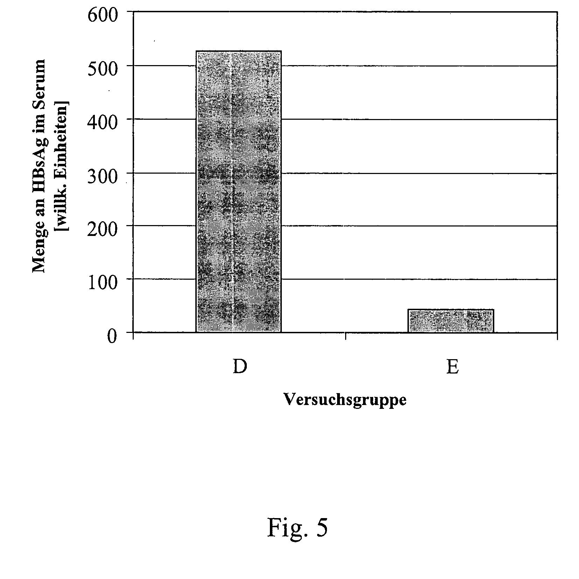

Wie den Ergebnissen in den Figuren 3 und 4 zu entnehmen ist, kann mit einer erfindungsgemäßen Zusammensetzung, welche einen heterologen HBsAg-Genotyp bzw. Subtyp beinhaltet (also ein HBsAg, welches auf einem Genotyp basiert, der sich von dem HBV-Genotyp der HBV-transgenen Maus unterscheidet) sowohl die Immuntoleranz gegen HBsAg als auch die Immuntoleranz gegen HBcAg durchbrochen werden. Insbesondere zeigt die Figur 5 (aus der hervorgeht, dass eine solche erfindungsgemäße Zusammensetzung zu einer Abnahme von HBsAg im Serum der HBV-transgenen Maus führt), dass eine solche erfindungsgemäße Zusammensetzung ein hohes therapeutisches Potential aufweist. Der in der Figur 5 beschriebene Effekt würde in einem an chronischer Hepatitis B leidenden Patienten auf eine Ausheilung hindeuten.

## Patentansprüche

1. Eine Zusammensetzung, beinhaltend:
i) ein HBcAg₁₋ₓ, wobei x eine ganze Zahl aus dem Bereich von 100 bis 160 ist, ein Fragment aus diesem Antigen, eine Variante dieses Antigens oder des Fragmentes dieses Antigens oder mindestens zwei davon
ii) ein Adjuvans beinhaltend ein Saponin,
sowie gegebenenfalls
iii) ein HBsAg, ein Fragment aus diesem Antigen, eine Variante dieses Antigens oder des Fragmentes dieses Antigens oder mindestens zwei davon,
wobei
- das Adjuvans als Saponin 50 bis 90 Gew.-% der gemäß der hierin beschriebenen Testmethode aus *Quillaja Saponaria* Molina-Extrakt erhaltenen Fraktion A und 10 bis 50 Gew.-% der gemäß der hierin beschriebenen Testmethode aus *Quillaja Saponaria* Molina-Extrakt erhaltenen Fraktion C enthält, wobei die Gesamtmenge aus Saponinen der Fraktion A und Saponinen der Fraktion C 100 Gew.-% beträgt;
- das Adjuvans neben dem Saponin Cholesterin, Phosphatidylcholin sowie Decanoyl-N-methylglucamid enthält.

2. Die Zusammensetzung nach Anspruch 1, wobei x eine ganze Zahl aus dem Bereich von 140 bis 149 ist.

3. Die Zusammensetzung nach Anspruch 1 oder 2, wobei x eine ganze Zahl aus dem Bereich von 144 bis 146 ist.

4. Die Zusammensetzung nach einem der vorhergehenden Ansprüche, wobei das Adjuvans in Form von Partikeln mit einem mittleren Partikeldurchmesser in einem Bereich von 20 bis 200 nm vorliegt.

5. Die Zusammensetzung nach Anspruch 4, wobei die einzelnen Adjuvans-Partikel sowohl Saponine der Fraktion A als auch Saponine der Fraktion C beinhalten.

6. Die Zusammensetzung nach einem der vorhergehenden Ansprüche, wobei das Adjuvans ein Saponinkomplex ist, der erhältlich ist durch ein Verfahren umfassend die folgenden Verfahrensschritte:
IIa) Bereitstellung von PBS-Lösung L₁ mit einem pH-Wert in einem Bereich von 7,2 bis 7,6;
IIb) Bereitstellung einer Lösung L₂ aus 5 bis 15 mg/ml Cholesterin und 5 bis 15 mg/ml Phosphatidylcholin in 10 bis 30 gew.-%iger wässriger Decanoyl-N-methylglucamid-Lösung;
IIc) Bereitstellung einer PBS-Lösung L₃ beinhaltend 50 bis 150 mg/ml der Mischung des Saponins aus den Fraktionen A und C mit einem pH-Wert in einem Bereich von 6,0 bis 6,4;
IId) Zugabe der Lösung L₂ zur Lösung L₁ im Volumenverhältnis L₂ : L₁ von 1 : 5 bis 1 : 15 und der Lösung L₃ zur Lösung L₁ im Volumenverhältnis L₃ : L₁ von 1 : 10 bis 1 : 30 und vermischen der so erhaltenen Lösung L₄;
IIe) Dialysieren der Lösung L₄ gegen PBS-Lösung mit einem pH-Wert in einem Bereich von 7,0 bis 7,8;
IIf) Dialysieren der Lösung L₄ gegen PBS-Lösung mit einem pH-Wert in einem Bereich von 6,0 bis 6,4;
IIg) gegebenenfalls nochmaliges Dialysieren der Lösung L₄ gegen PBS-Lösung mit einem pH-Wert in einem Bereich von 6,0 bis 6,4.

7. Die Zusammensetzung nach einem der vorhergehenden Ansprüche, wobei das relative Mengenverhältnis zwischen der Antigenkomponente i) bzw. den Antigenkomponenten i) und iii) zum Adjuvans ii) in einem Bereich von 1 : 20 bis 20 : 1 liegt.

8. Die Zusammensetzung nach einem der vorhergehenden Ansprüche, wobei die Gesamtkonzentration der Komponenten i), ii) und gegebenenfalls iii) in der Zusammensetzung in einem Bereich von 0,1 bis 2.000 µg/ml liegt.

9. Die Zusammensetzung nach Anspruch 1, umfassend
i) ein HBcAg₁₋₁₄₅ sowie
ii) ein Adjuvans, wie in einem der Ansprüche 4 bis 6 definiert.

10. Die Zusammensetzung nach Anspruch 1, umfassend
i) ein HBcAg_{1-144+I} sowie
ii) ein Adjuvans, wie in einem der Ansprüche 4 bis 6 definiert.

11. Die Zusammensetzung nach Anspruch 1, umfassend
i) ein HBcAg₁₋₁₄₅,
ii) ein Adjuvans, wie in einem der Ansprüche 4 bis 6 definiert, sowie
iii) ein HBsAg.

12. Die Zusammensetzung nach Anspruch 1, umfassend
i) ein HBcAg_{1-144+I},
ii) ein Adjuvans, wie in einem der Ansprüche 4 bis 6 definiert, sowie
iii) ein HBsAg.

13. Ein Verfahren zur Herstellung einer Zusammensetzung, umfassend die Verfahrensschritte I), II) und IV) und gegebenenfalls III):
I) Bereitstellen eines HBcAg₁₋ₓ's, wobei x eine ganze Zahl aus dem Bereich von 100 bis 160 ist, eines Fragmentes aus diesem Antigen, einer Variante dieses Antigens oder des Fragmentes dieses Antigens oder einer Mischung aus mindestens zwei davon,
II) Bereitstellen eines Adjuvans beinhaltend ein Saponin,
wobei
- das Adjuvans als Saponin 50 bis 90 Gew.-% der gemäß der hierin beschriebenen Testmethode aus *Quillaja Saponaria* Molina-Extrakt erhaltenen Fraktion A und 10 bis 50 Gew.-% der gemäß der hierin beschriebenen Testmethode aus *Quillaja Saponaria* Molina-Extrakt erhaltenen Fraktion C enthält, wobei die Gesamtmenge aus Saponinen der Fraktion A und Saponinen der Fraktion C 100 Gew.-% beträgt;
- das Adjuvans neben dem Saponin Cholesterin, Phosphatidylcholin sowie Decanoyl-N-methylglucamid enthält,
III) Bereitstellen eines HBsAg's, eines Fragmentes aus diesem Antigen, einer Variante dieses Antigens oder des Fragmentes dieses Antigens oder einer Mischung aus mindestens zwei davon, und
IV) In Kontakt bringen des HBcAg₁₋ₓ's, des Adjuvans und gegebenenfalls des HBsAg's.

14. Das Verfahren nach Anspruch 13, wobei x eine ganze Zahl aus dem Bereich von 140 bis 149 ist.

15. Das Verfahren nach Anspruch 13 oder 14, wobei x eine ganze Zahl aus dem Bereich von 144 bis 146 ist.

16. Das Verfahren nach einem der Ansprüche 13 bis 15, wobei das Adjuvans ein Saponinkomplex ist, der erhältlich ist durch ein Verfahren umfassend die Verfahrensschritte:
IIa) Bereitstellung von PBS-Lösung L₁ mit einem pH-Wert in einem Bereich von 7,2 bis 7,6;
IIb) Bereitstellung einer Lösung L₂ aus 5 bis 15 mg/ml Cholesterin und 5 bis 15 mg/ml Phosphatidylcholin in 10 bis 30 gew.-%iger wässriger Decanoyl-N-methylglucamid-Lösung;
IIc) Bereitstellung einer PBS-Lösung L₃ beinhaltend 50 bis 150 mg/ml der Mischung des Saponins aus den Fraktionen A und C mit einem pH-Wert in einem Bereich von 6,0 bis 6,4;
IId) Zugabe der Lösung L₂ zur Lösung L₁ im Volumenverhältnis L₂ : L₁ von 1 : 5 bis 1 : 15 und der Lösung L₃ zur Lösung L₁ im Volumenverhältnis L₃ : L₁ von 1 : 10 bis 1 : 30 und vermischen der so erhaltenen Lösung L₄;
IIe) Dialysieren der Lösung L₄ gegen PBS-Lösung mit einem pH-Wert in einem Bereich von 7,0 bis 7,8;
IIf) Dialysieren der Lösung L₄ gegen PBS-Lösung mit einem pH-Wert in einem Bereich von 6,0 bis 6,4;
IIg) gegebenenfalls nochmaliges Dialysieren der Lösung L₄ gegen PBS-Lösung mit einem pH-Wert in einem Bereich von 6,0 bis 6,4.

17. Eine Zusammensetzung, erhältlich durch ein Verfahren nach einem der Ansprüche 13 bis 16.

18. Eine Arzneimittelformulierung, umfassend die Zusammensetzung nach einem der Ansprüche 1 bis 12 oder 17 sowie geeignete Zusatzstoffe.

19. Die Verwendung der Zusammensetzung nach einem der Ansprüche 1 bis 12 oder 17 zur Herstellung einer Arzneimittelformulierung zur Therapie und/oder zur Prophylaxe von HBV-Infektionen und HBV-vermittelten Erkrankungen.

20. Die Verwendung der Zusammensetzung nach einem der Ansprüche 1 bis 12 oder 17 zur Herstellung einer Arzneimittelformulierung zur Behandlung chronischer HBV-Infektionen.

21. Die Verwendung nach Anspruch 19 oder 20, wobei die Zusammensetzung das HBsAg umfasst und wobei die HBV-Infektion oder die HBV-vermittelte Erkrankung durch ein HB-Virus verursacht worden sind, dessen Genotyp sich vom Genotyp des in der Zusammensetzung enthaltenen HBsAg's unterscheidet.


SEQUENCE LISTING

<110>  Rhein Biotech Gesellschaft für neue biotechnologische Prozesse und
Produkte mbH

<120>  Eine Zusammensetzung zur Therapie und/oder Prophylaxe von HBV-Infektionen
 und HBV-vermittelten Erkrankungen

<130>  RB83434

<140>  EP 06019910.6

<141>  2006-09-22

<160>  3

<170>  PatentIn version 3.1

<210>  1

<211>  8

<212>  PRT

<213>  künstliche Sequenz

<220>

<221>  PEPTIDE

<222>  (1)..(8)

<223>  HBcAg-spezifisches Peptid

<400>  1

Met Gly Leu Lys Phe Arg Gln Leu
1               5

<210>  2

<211>  8

<212>  PRT

<213>  künstliche Sequenz

<220>

<221>  PEPTIDE

<222>  (1)..(8)

<223>  HBsAg-spezifisches Peptid


<400>  2

Val Trp Leu Ser Val Ile Trp Met
1               5


<210>  3

<211>  23

<212>  DNA

<213>  künstliche Sequenz


<220>

<221>  misc_structure

<222>  (1)..(23)

<223>  synthetischer Polylinker


<400>  3
tccggagata tcgatcgtcc gga                                              23

SEQUENCE LISTING

<110>  Rhein Biotech Gesellschaft für neue biotechnologische Prozesse und
Produkte mbH

<120>  Eine Zusammensetzung zur Therapie und/oder Prophylaxe von HBV-Infektionen
 und HBV-vermittelten Erkrankungen

<130>  RB83434

<160>  3

<170>  PatentIn version 3.1

<210>  1
<211>  8
<212>  PRT
<213>  synthetic polypeptide

<400>  1

Met Gly Leu Lys Phe Arg Gln Leu
1               5

<210>  2
<211>  8
<212>  PRT
<213>  synthetic polypeptide

<400>  2

Val Trp Leu Ser Val Ile Trp Met
1               5

<210>  3
<211>  23
<212>  DNA
<213>  synthetic polynucleotide

<400> 3
tccggagata tcgatcgtcc gga 23